# EUROPÄISCHE PATENTSCHRIFT

(11) **EP 1 572 640 B1**
(45) Veröffentlichungstag und Bekanntmachung des Hinweises auf die Patenterteilung: **07.11.2012**
(21) Anmeldenummer: 03780043.0
(22) Anmeldetag: 25.11.2003
(51) Int. Cl.: C07D 207/12, A61K 31/40

(54) **DERIVATE DES ASIMADOLINS MIT KOVALENT GEBUNDENEN SAEUREN**
ASIMADOLINE DERIVATIVES COMPRISING COVALENTLY BONDED ACIDS
DERIVES DE L'ASIMADOLINE COMPORTANT DES ACIDES LIES PAR COVALENCE

(30) Priorität: 17.12.2002 DE 10259245
(43) Veröffentlichungstag der Anmeldung: 14.09.2005
(73) Patentinhaber: Tioga Pharmaceuticals, Inc., San Diego, CA 92121 (US)
(72) Erfinder: WIESNER, Matthias, 55128 Mainz (DE); SEYFRIED, Christoph, 64342 Seeheim-Jugenheim (DE)
(74) Vertreter: Viering, Jentschura & Partner
(86) Internationale Anmeldenummer: PCT/EP2003/013206
(87) Internationale Veröffentlichungsnummer: WO 2004/054970

(56) Entgegenhaltungen:
- EP-A- 0 569 802
- WO-A-00/14065

## Beschreibung

Die vorliegende Erfindung betrifft Derivate von N-Methyl-N-[(1S)-1-phenyl-2-((3S)-3-hydroxypyrrolidin-1-yl)-ethyl]-2,2-diphenyl-acetamid mit kovalent gebundenen Säuren, diese Derivate als Arzneimittel, die Verwendung dieser Derivate für die Herstellung eines Arzneimittels, die Verwendung dieser Derivate zur Herstellung einer pharmazeutischen Zusammensetzung, ein Verfahren zur Herstellung besagter pharmazeutischer Zusammensetzungen, pharmazeutische Zusammensetzungen, erhältlich durch dieses Verfahren .

Die Verbindung N-Methyl-N-[(1S)-1-phenyl-2-((3S)-3-hydroxypyrrolidin-1-yl)-ethyl]-2,2-diphenyl-acetamid und insbesondere das Hydrochlorid (EMD 61753) ist bekannt, z. B. unter dem Namen Asimadolin oder Asimadoline, und wurde bereits in der EP-A-0 569 802 (US 5,532,226), der EP-A-0 752 246 (US 5,776,972 und US 5,977,161), DE-A-198 49 650, EP-A-0 761 650 (US 6,060,504) und der EP-A-1 073 634 (US 6,344,566) beschrieben. Das Hydrochlorid dieser Verbindung kann als Arzneimittelwirkstoff eingesetzt werden und zeigt eine Reihe vorteilhafter Eigenschaften bei verschiedenen Indikationen.

Der Erfindung lag die Aufgabe zu Grunde, neue stabile Derivate von N-Methyl-N-[(1S)-1-phenyl-2-((3S)-3-hydroxypyrrolidin-1-yl)-ethyl]-2,2-diphenyl-acetamid zu finden, die eine bessere Löslichkeit, bessere Resorption, größere Stabilität, geringere Hygroskopie und/oder verbesserte pharmakokinetische Eigenschaften aufweisen.

Überraschend wurden nun neue Derivate von N-Methyl-N-[(1S)-1-phenyl-2-((3S)-3-hydroxypyrrolidin-1-yl)-ethyl]-2,2-diphenyl-acetamid gefunden, die gegenüber den bekannten Darreichungsformen, insbesondere gegenüber N-Methyl-N-[(9S)-1-phenyl-2-((3S)-3-hydroxypyrrolidin-1-yl)-ethyl]-2,2-diphenyl-acetamid, Hydrochlorid, als Arzneimittelwirkstoff für eine Vielzahl von Indikationen vorteilhafte Eigenschaften zeigen: Zu den vorteilhaften Eigenschaften der erfindungsgemäßen Derivate gehören vorzugsweise ein verbessertes Löslichkeitsverhalten, ein modifiziertes, insbesondere verbessertes pharmakokinetisches Verhalten, ein modifiziertes Verträglichkeitsprofil und/oder eine modifizierte Halbwertszeit, vorzugsweise eine verlängerte Halbwertszeit.

Überraschenderweise wechselwirken die erfindungsgemäßen Derivate intensiv mit dem enterohepatischen Kreislauf. So können erfindungsgemäße Derivate durch Wechselwirkungen mit dem enterohepatischen Kreislauf gespalten, derivatisiert und/oder zusätzlich metabolisiert werden oder aus freiem N-Methyl-N-[(1S)-1-phenyl-2-((3S)-3-hydroxypyrrolidin-1-yl)-ethyl]-2,2-diphenyl-acetamid gebildet werden. Ferner kann ein erfindungsgemäßes Derivat durch Wechselwirkungen mit dem enterohepatischen Kreislauf in ein anderes erfindungsgemäßes Derivat überführt werden. Es wird vermutet, das wenigstens ein Teil der vorteilhaften Eigenschaften der erfindungsgemäßen Derivate durch die Wechselwirkungen zwischen erfindungsgemäßen Derivat enterohepatischen Kreislauf zu Stande kommt.

Gegenstand der vorliegenden Erfindung sind daher Derivate von N-Methyl-N-[(1S)-1-phenyl-2-((3S)-3-hydroxypyrrolidin-1-yl)-ethyl]-2,2-diphenyl-acetamid mit wenigstens einer kovalent gebundenen Säure wobei die Saüre ausgewählt wird aus zweibasigen Carbonsaüren, einbasigen Hydroxycarbonsaüren und wenigstens zweibasigen anorganischen Sauerstoffsaüren. Ebenfalls Gegenstand dieser Erfindung sind die Salze, Solvate und Prodrugs der Derivate von N-Methyl-N-[(1S)-1-phenyl-2-((3S)-3-hydroxypyrrolidin-1-yl)-ethyl]-2,2-diphenyl-acetamid mit wenigstens einer kovalent gebundenen Säure. Kovalent gebundene Säure im Sinne der Erfindung bedeutet, dass das Derivat von N-Methyl-N-[(1S)-1-phenyl-2-((3S)-3-hydroxypyrrolidin-1-yl)-ethyl]-2,2-diphenyl-acetamid wenigstens eine Säure bzw. einen von einer Säure abgeleiteten Rest aufweist, der nicht durch ionische Wechselwirkung, insbesondere nicht durch Salzbildung, mit dem N-Methyl-N-[(1S)-1-pheny(-2-((3S)-3-hydroxypyrrolidin-1-yl)-ethyl]-2,2-d iphenyl-acetamid verbunden ist. Vorzugsweise umfasst das erfindungsgemäße Derivat wenigstens eine Säure bzw. einen von einer Säure abgeleiteten Rest, der durch Veresterung oder Veretherung mit dem N-Methyl-N-[(1S)-1-phenyl-2-((3S)-3-hydroxypyrrolidin-1-yl)-ethyl]-2,2-diphenyl-acetamid verbunden ist.

Die Säure bzw. der von einer Säure abgeleitete Rest ist über die 3-Hydroxypyrrolidin-Gruppe des N-Methyl-N-[(1S)-1-phenyl-2-((3S)-3-hydroxypyrrolidin-1-yl)-ethyl]-2,2-diphenyl-acetamids an das N-Methyl-N-[(1S)-1-phenyl-2-((3S)-3-hydroxypyrrolidin-1-yl)-ethyl]-2,2-diphenyl-acetamid gebunden.

Bei den erfindungsgemäßen Derivaten, ist daher die Säure bzw. der von einer Säure abgeleitete Rest.durch Veresterung oder Veretherung mit der 3-Hydroxypyrrolidin-Gruppe kovalent an das N-Methyl-N-[(1S)-1-phenyl-2-((3S)-3-hydroxypyrrolidin-1-yl)-ethyl]-2,2-diphenyl-acetamid gebunden . N-Methyl-N-[(1S)-1-phenyl-2-((3S)-3-hydroxypyrrolidin-1-yl)-ethyl]-2,2-diphenyl-acetamid weist eine freie Hydroxy-Funktion in der 3-Position der Pyrrolidin-Gruppe auf und ist daher ein Alkohol. Bevorzugt als erfindungsgemäße Derivate sind daher die Ester oder Ether des N-Methyl-N-[(1S)-1-phenyl-2-((3S)-3-hydroxypyrrolidin-1-yl)-ethyl]-2,2-diphenyl-acetamids, und ganz besonders bevorzugt die Ester oder Ether des N-Methyl-N-[(1S)-1-phenyl-2-((3S)-3-hydroxypyrrolidin-1-yl)-ethyl]-2,2-diphenyl-acetamids mit einer der aufgeführten Säuren.

Anorganische Sauerstoffsäuren schließen schweflige Säure, Schwefelsäure, und Phosphorsäuren, bevorzugt Orthophosphorsäure ein Zweibasige Carbonsäuren und ein basige Hydroxycarbonsäuren Schließen ein: Malonsäure, Bernsteinsäure, Pimelinsäure, Fumarsäure, Maleinsäure, Milchsäure, Gluconsäure, Glucuronsäure, Galacturonsäure und Ascorbinsäure

Vorzugsweise ist die Säure ausgewählt unter physiologisch verträglichen Säuren, insbesondere unter den physiologisch verträglichen Säuren der vorstehend genannten Säuren.

Vorzugsweise sind die Carbonsäuren ausgewählt unter zweibasigen Carbonsäuren, mit 1 bis 12 Kohlenstoffatomen, bevorzugt 1 bis 6 Kohlenstoffatomen, wie den Dicarbonsäuren Malonsäure, Bernsteinsäure, Pimelinsäure, Fumarsäure und Mareinsäure.

Vorzugsweise sind die Hydroxycarbonsäuren ausgewählt unter Monohydroxymonocarbonsäuren, wie Milchsäure, und Polyhydroxymonocarbonsäuren, wie Zuckersäuren, insbesondere Ascorbinsäure, Glucuronsäure und Galacturonsäure.

Vorzugsweise sind die anorganischen Sauerstoffsäuren ausgewählt unter Schwefelsäure und Orthophosphorsäure und insbesondere Schwefelsäure.

Besonders bevorzugt sind erfindungsgemäße Derivate, die wenigstens eine freie Säuregruppe, das heißt eine zur Salzbildung befähigte oder eine als Salz vorliegende Säurefunktion, aufweisen.

Die vorliegende Erfindung betrifft daher erfindungsgemäße Derivate, worin die Säure ausgewählt ist unter zweibasigen Carbonsäuren, einbasigen Hydroxycarbonsäuren und wenigstens zweibasigen anorganischen Sauerstoffsäuren, insbesondere unter den vorstehend als bevorzugt genannten zweibasigen Carbonsäuren, einbasigen Hydroxycarbonsäuren und wenigstens zweibasigen anorganischen Sauerstoffsäuren.

Eine besonders bevorzugte Ausführungsform der vorliegenden Erfindung betrifft erfindungsgemäße Derivate, worin die einbasige Hydroxycarbonsäure unter Zuckersäuren ausgewählt ist und insbesondere Glucuronsäure ist.

Eine weitere besonders bevorzugte Ausführungsform der vorliegenden Erfindung betrifft erfindungsgemäße Derivate, worin die zweibasige anorganische Sauerstoffsäure Schwefelsäure ist.

Eine besonders bevorzugte Ausführungsform der vorliegenden Erfindung betrifft erfindungsgemäße Derivate, worin die Säure ausgewählt ist unter Glucuronsäure und Schwefelsäure.

Bevorzugt die erfindungsgemäßen Derivate ausgewählt unter den Estern der vorstehend genannten Monocarbonsäuren mit N-Methyl-N-[(1S)-1-phenyl-2-((3S)-3-hydroxypyrrolidin-1-yl)-ethyl]-2,2-diphenyl-acetamid als Alkohol-Komponente, den Estern der vorstehend genannten anorganischen Sauerstoffsäuren mit N-Methyl-N-[(1S)-1-phenyl-2-((3S)-3-hydroxypyrrolidin-1-yl)-ethyl]-2,2-diphenyl-acetamid als Alkohol-Komponente und den Ethern der vorstehend genannten Hydroxycarbonsäuren mit N-Methyl-N-[(1S)-1-phenyl-2-((3S)-3-hydroxypyrrolidin-1-yl)-ethyl]-2,2-diphenyl-acetamid als zweiter Alkohol-Komponente.

Bei erfindungsgemäßen Estern mehrbasiger Säuren sind in der Regel die Monoester bevorzugt. Bei erfindungsgemäßen Ethern von Polyhydroxycarbonsäuren sind in der Regel die Monoether bevorzugt. In der Regel liegen die erfindungsgemäßen Derivate von N-Methyl-N-[(1S)-1-phenyl-2-((3S)-3-hydroxypyrrolidin-1-yl)-ethyl]-2,2-diphenyl-acetamid und Hydroxycarbonsäuren im wesentlichen weder als gemischte Ester und Ether einer Hydroxycarbonsäure vor, noch als Gemisch zweier Derivate, worin ein Teil des N-Methyl-N-[(1S)-1-phenyl-2-((3S)-3-hydroxypyrrolidin-1-yl)-ethyl]-2,2-diphenyl-acetamids mit der Hydroxycarbonsäure in Form eines Ethers verbunden ist und ein Teil N-Methyl-N-[(1S)-1-phenyl-2-((3S)-3-hydroxypyrrolidin-1-yl)-ethyl]-2,2-diphenyl-acetamids mit der Hydroxycarbonsäure in Form eines Esters verbunden ist.

Daher sind erfindungsgemäße Derivate besonders bevorzugt, die zu wenigstens 40 Gew.%, vorzugsweise zu wenigstens 60 Gew.-%, besonders bevorzugt zu wenigstens 70 Gew.-%, ganz besonders bevorzugt zu wenigstens 80 Gew.% oder wenigstens 90 Gew.-%, maximal zu 100 Gew.-%, aber in vielen Fällen zu weniger als 100 Gew.-%, beispielsweise zu 60 bis 90 Gew.-%, 70 bis 95 Gew.-%, 80 bis 99.9 Gew.-% oder 90 bis 100 Gew.%, aus einer einzelnen, definierten Verbindung bestehen, die vorzugsweise nur ein Molekül bzw. eine Einheit von N-Methyl-N-[(1S)-1-phenyl-2-((3S)-3-hydroxypyrrolidin-1-yl)-ethyl]-2,2-diphenyl-acetamid als Alkohol-Komponente umfassen und ein Molekül bzw. eine Einheit einer Säure, vorzugsweise einer vorstehend genannten Säure, kovalent gebunden enthalten.

Beispiele für bevorzugte erfindungsgemäße Derivate sind Verbindungen der Formel I 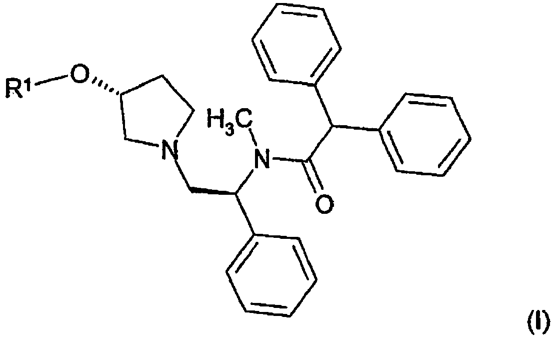 worin R¹ ausgewählt ist unter
a) Acylresten mit 1 bis 12, vorzugsweise 1 bis 6 Kohlenstoffatomen, die eine Hydroxy-Gruppe oder eine Carboxylgruppe aufweisen, und besonders bevorzugt ausgewählt unter 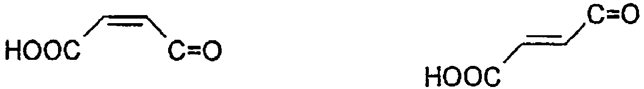 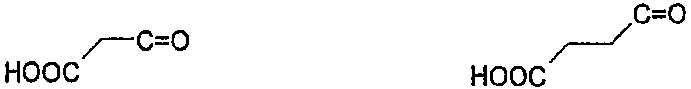 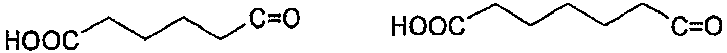 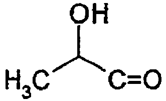
b) Alkylresten, die durch Entfernung einer Hydroxygruppe von Polyhydroxymonocarbonsäuren abgeleitet sind, vorzugsweise von Ascorbinsäure, Glucuronsäure und Galacturonsäure, besonders bevorzugt 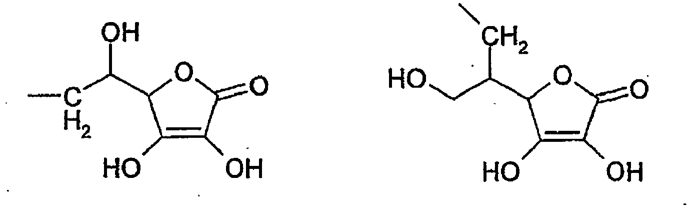 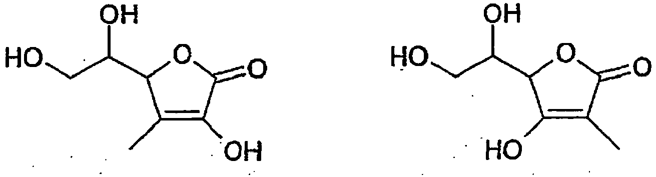 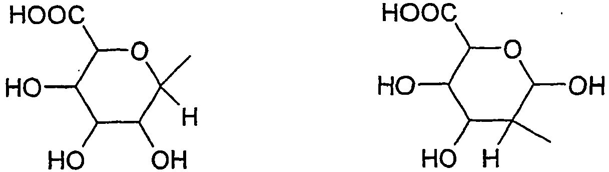 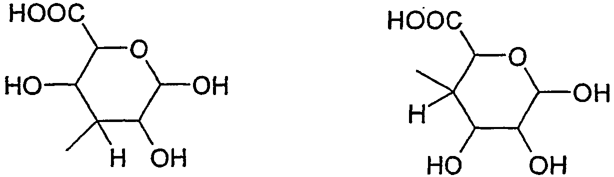 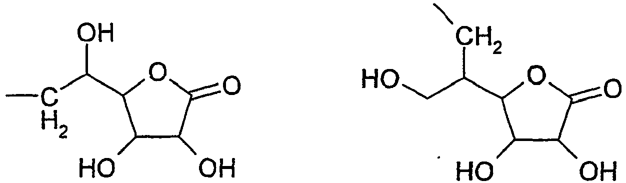 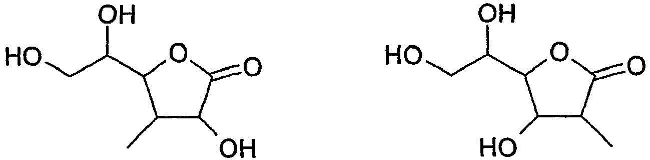 ganz besonders bevorzugt 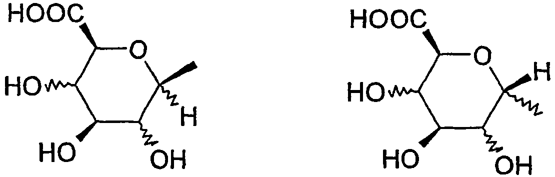 und insbesondere 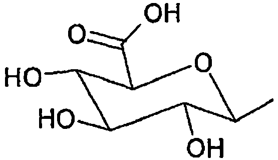 und/oder den offenkettigen Formen davon;
   sowie unter
c) Sulfonsäuregruppen, die durch Entfernung einer Hydroxygruppe von Schwefelsäure abgeleitet sind und Phosphonsäuregruppen, die durch Entfernung einer Hydroxygruppe von Orthophosphorsäure abgeleitet sind, insbesondere 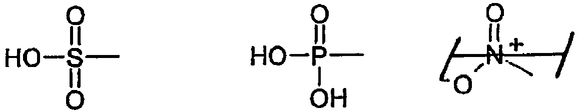 sowie die Salze und Solvate davon.

In den unter b) und c) wiedergegebenen Resten R¹ symbolisiert die freie Bindung bzw. der freie Valenzstrich aus Klarheitsgründen vorzugsweise die Stelle, mit der R¹ in der Formel I an das Sauerstoffatom bindet. in denen unter b) wiedergegebenen Resten R¹ symbolisiert der ausgefüllte Keil vorzugsweise eine Bindung, die gegenüber der Papierebene nach oben zeigt. In denen unter b) wiedergegebenen Resten R¹ symbolisiert der schraffierte Keil vorzugsweise eine Bindung, die gegenüber der Papierebene nach unten zeigt.

Besonders bevorzugte erfindungsgemäße Derivate sind ausgewählt unter Verbindungen der Formel la 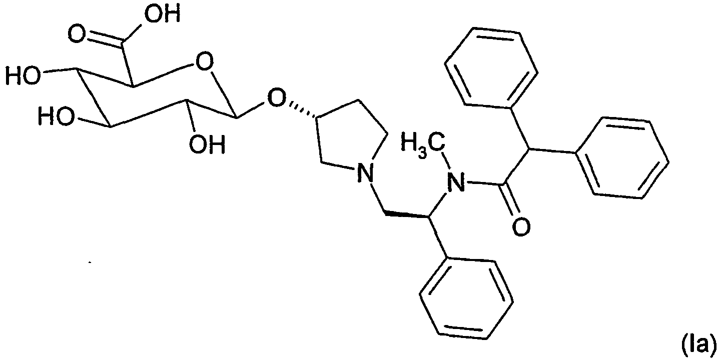 (6-((1S)-1-{[(2,2-Diphenyl-ethanoyl)-methyl-amino]-phenyl-ethyl}-(3S)-pyrrolidin-3-yloxy)-D-glucuronsäure)
und Verbindungen der Formel Ib 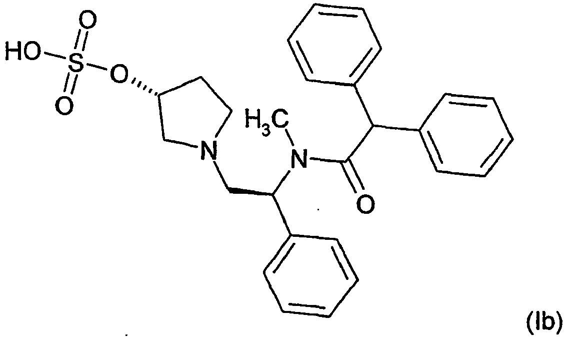 (Schwefelsäuremono-((1S)-1-{[(2,2-diphenyl-ethanoyl)-methyl-amino]-phenyl-ethyl}-(3S)-pyrrolidin-3-yl)ester)
sowie den Salzen und Solvaten davon.

Gegenstand der Erfindung sind auch pharmazeutisch verträgliche Derivate der erfindungsgemäßen Verbindungen, insbesondere Prodrugs bzw. Prodrug-Derivate, Salze und Solvate der erfindungsgemäßen Verbindungen, insbesondere der Verbindungen der Formel I und deren Subformeln.

Gegenstand der Erfindung sind auch die optisch aktiven Formen (Stereoisomere), vorzugsweise die Enantiomeren, die Racemate, die Diastereomeren sowie die Hydrate und Solvate der erfindungsgemäßen Verbindungen. Unter Solvaten der erfindungsgemäßen Verbindungen werden Anlagerungen von inerten Lösungsmittelmolekülen an die erfindungsgemäßen Verbindungen verstanden, die sich aufgrund ihrer gegenseitigen Anziehungskraft ausbilden. Solvate sind z.B. Mono- oder Dihydrate oder Alkoholate.

Unter Prodrugs von erfindungsgemäßen Verbindungen versteht man die Alkylester von erfindungsgemäßen Derivaten, die wenigstens eine freie Säuregruppe aufweisen, oder die Ester von erfindungsgemäßen Derivaten, die wenigstens eine freie Hydroxy-Gruppe aufweisen.

Gegenstand der Erfindung sind auch Mischungen der erfindungsgemäßen Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Unter den pharmazeutisch verträglichen Derivaten sind die Salze und Solvate von erfindungsgemäßen Derivaten, insbesondere den erfindungsgemäßen Derivaten der Formel I, und insbesondere die physiologisch verträglichen Salze und Solvate davon bevorzugt.

Ein erfindungsgemäßes Derivat und insbesondere eine Verbindung der Formel I kann durch Einwirkung einer Säure oder einer Base in ein Salz überführt werden. So kann ein erfindungsgemäßes Derivat, insbesondere ein basisches erfindungsgemäßes Derivat, mit einer Säure in das zugehörige Säureadditionssalz übergeführt werden, beispielsweise durch Umsetzung äquivalenter Mengen der Base und der Säure in einem inerten Lösungsmittel, wie Ethanol, und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und/oder Aufreinigung der erfindungsgemäßen Derivate verwendet werden.

Die zur Bildung des Säureadditionssalzes eingesetzte Säure kann gleich oder verschiedenen von der im erfindungsgemäßen Derivate kovalent gebundenen Säure sein, jedoch liegt die zur Bildung des Säureadditionssalzes eingesetzte Säure im Salz (Säureadditionssalz) nicht kovalent gebunden, sondern durch ionische Wechselwirkungen assoziiert vor. Vorzugsweise ist jedoch die im erfindungsgemäßen Derivat kovalent gebundenen Säure von der zur Bildung des Säureadditionssalzes verwendeten Säure verschieden. Sofern ein erfindungsgemäßes Derivat als Salz und insbesondere als physiologisch verträgliches Salz vorliegt, handelt es sich dabei in der Regel um Säureadditionssalz und insbesondere um ein physiologisch verträgliches Säureadditionssalz.

Wenn die im erfindungsgemäßen Derivat kovalent gebundene Säure eine oder mehrere freie Säuregruppen aufweist, kann das erfindungsgemäße Derivat auch ohne Zugabe einer Säure oder Base durch interne Salzbildung als so genanntes inneres Salz in der Salzform vorliegen.

Wenn die im erfindungsgemäßen Derivat kovalent gebundene Säure eine physiologisch verträgliches Säure ist und eine oder mehrere freie Säuregruppen aufweist, kann das erfindungsgemäße Derivat auch ohne Zugabe einer Säure oder Base durch interne Salzbildung als physiologisch verträgliches Salz vorliegen.

Alternativ kann ein erfindungsgemäßes Derivat, insbesondere ein saures erfindungsgemäßes Derivat, mit einer Base in das Baseadditionssalz überführt werden, beispielsweise durch Umsetzung äquivalenter Mengen eines erfindungsgemäßen Derivats und einer Base in einem inerten Lösungsmittel, wie Ethanol, und anschließendes Eindampfen. Für diese Umsetzung kommen insbesondere Basen in Frage, die physiologisch unbedenkliche Salze liefern. Geeignete Basen sind dem Fachmann bekannt. Beispielsweise können erfindungsgemäße Derivate mit Basen, wie Aminen, Alkalimetallhydroxiden, Erdalkalimetallhydroxiden, Alkalimetallcarbonaten und Erdalkalimetallcarbonaten, vorzugsweise Natrium- oder Kaliumhydroxid, Natrium- oder Kaliumcarbonat, in die entsprechenden Metall-, insbesondere Alkalimetall- oder Erdalkalimetall-, oder in die entsprechenden Ammoniumsalze umgewandelt werden. Auch physiologisch unbedenkliche organische Basen, wie z.B. Ethanolamin können verwendet werden.

Bevorzugter Gegenstand der vorliegenden Erfindung sind die erfindungsgemäßen Derivate in isolierter und/oder im wesentlichen reiner Form. Im wesentlichen rein bedeutet vorzugsweise, dass die erfindungsgemäßen Derivate in einer Reinheit von größer als 60 Gew.-%, bevorzugt größer als 80 Gew.-%, besonders bevorzugt größer als 90 Gew.-% und insbesondere größer als 95 Gew.-%, wie beispielsweise größer als 97 Gew.-%, größer als 99 Gew.-%, größer als 99,9 Gew.-%, größer als 99,99 Gew.-%, oder eine Reinheit von bis zu 100 Gew.-% aufweisen. Bei Gemischen erfindungsgemäßer Derivate beziehen sich die Reinheitsangaben vorzugsweise auf das jeweilige Derivat und insbesondere auf die als Hauptbestandteile enthaltenden erfindungsgemäßen Derivate. Unter Hauptbestandteile in diesem Zusammenhang werden vorzugsweise die im Gemisch enthaltenen erfindungsgemäßen Derivate bezeichnet, die im größten Anteil enthalten sind, wobei der Anteil eines Hauptbestandteils am Gemisch vorzugsweise wenigstens 10 Gew.%, bevorzugt wenigstens 30 Gew.-%, besonders bevorzugt wenigstens 60 Gew.-% und insbesondere wenigstens 80 Gew.-%, aber weniger als 100 Gew.%, bezogen auf das Gesamtgewicht der enthaltenen erfindungsgemäßen Derivate, beträgt.

Die erfindungsgemäßen Derivate und/oder die Salze und Solvate davon zeigen eine Reihe wertvoller pharmakologischer Eigenschaften. So zeigen sie vorzugsweise analgetische, neuroprotektive, antiinflamatorische, antiasthmatische, diuretische, antikonvulsive und/oder antitussive Wirkungen, antagonisieren Hyperalgesie, insbesondere entzündungsbedingte Hyperalgesie, schützen vor und eignen sich zur Behandlung von Schmerzzuständen, Himödemen, Unterversorgungszuständen des Zentralnervensystems und insbesondere Hypoxie, und zur Behandlung und Verminderung der Folgeschäden nach Ischämien, wie beispielsweise in der EP-A-0 569 802 beschrieben. So haben Versuche gezeigt, dass die erfindungsgemäßen Verbindungen im "Writhing Test" an Mäusen oder Ratten wirken (Methode vgl. Siegmund et. al., Proc. Soc. Exp. Biol. 95, (1957), 729-731). Die analgetische Wirkung als solche lässt sich ferner im "Tail-Flick-Test" an Mäusen oder Ratten nachweisen (Methodik vgl. d'Amour and Smith, J. Pharmacol. Exp. Ther. 72, (1941), 74-79), ferner im "Hot plate test" (vgl. Schmauss und Yaksh, J. Pharmacol. Exp. Ther. 228, (1984), 1-12 und die dort zitierte Literatur). Besonders starke Wirkungen sind an Ratten im Modell der Carrageenin-induzierten Hyperalgesie (vgl. Bartoszyk und Wild, Neuroscience Letters 101 (1989) 95) zu beobachten. Dabei zeigen die Verbindungen keine oder nur geringe Neigung zu physischer Abhängigkeit. Außerdem wurden durch entsprechende, nach geläufigen Methoden durchgeführte Versuche die ausgeprägten antiinflammatorischen, diuretischen, antikonvulsiven, neuroprotektiven Wirkungen nachgewiesen. Die erfindungsgemäßen Derivate zeigen eine hohe Affinität in bezug auf das Bindungsverhalten an kappa-Rezeptoren.

Ein besonderer Aspekt der vorliegende Erfindung betrifft die Wirksamkeit der erfindungsgemäßen Derivate zur Prophylaxe und/oder Behandlung funktioneller gastrointestinaler Erkrankungen, im Folgenden auch funktionelle Magen-und/oder Darmerkrankungen oder kurz funktionelle Magen-Darmerkrankungen genannt. Die Gruppe der funktionellen gastrointestinalen Erkrankungen ist ausführlich beschrieben und klassifiziert in GUT: Rome II: A Multinational consensus Document on Functional Gastrointestinal Disorders, Supplement 11, Vol 45, 1999

Vorzugsweise sind die funktionellen gastrointestinalen Erkrankungen ausgewählt unter funktionellen gastroduodenalen Erkrankungen, darunter bevorzugt funktionelles Erbrechen und besonders bevorzugt Dyspepsien, insbesondere nicht mit einem Ulcus verbundene Dyspepsien; funktionellen Darmerkrankungen, darunter vorzugsweise funktionelle Abdominalschmerzen, bevorzugt funktionelle Blähungen bzw. Flatulenz, besonders bevorzugt funktionelle Obstipation, Konstipation oder Verstopfung, ebenfalls besonders bevorzugt funktionelle Diarrhoe, und insbesondere Reizdarm, irritabler Colon bzw. IBS (Irritable Bowel Syndrome); und chronischen Motilitätsstörungen.

Die Wirksamkeit der erfindungsgemäßen Derivate in den genannten Indikationen bzw. Krankheitsbildern kann mit gängigen, aus dem Stand der Technik bekannten Verfahren oder dazu analogen Verfahren nachgewiesen werden. Die Indikationen und Verfahren zum Nachweis der Wirksamkeit in diesen Indikationen sind beispielsweise beschrieben in N. J. Talley, V. Stanghellini, R. C. Heading, K. L. Koch, J. R. Malagelada, G. N. J. Tytgat; Gut 1999; 45 (Suppl 2): II37-II42; W. G. Thompson, G. F. Longstreth, D. A. Drossman, K. W. Heaton, E. J. Irvine, S. A. Müller-Lissner; Gut 1999; 45 (Suppl 2): II43-II47;S. J. O. Veldhuyzen van Zanten, N. J. Talley, P. Bytzer, K. B. Klein, P. J. Whorwell, A. R. Zinsmeister; Gut 1999; 45 (Suppl 2):II169-II77; M. Dapoigny, M. Homerin, B. Scherrer, B. Fraitag; Gut (34, SuppL. 3, S30, 1993); J.-L. Abitbol, B. Scherrer, C. de Meynard, G. Meric, B. Fraitag, Gut (39, Suppl. 3, A229-A230, 1996); and N. J. Talley, S. V. Van Zanten, L. R. Saez, G. Dukes, T. Perschy, M. Heath, C. Kleoudis, A. W. Mangel; Alimentary Pharmacology and Therapeutics 15: 4, 525-537.

Daher betrifft die vorliegende Erfindung auch die erfindungsgemäßen Derivate zur Diagnose, Prophylaxe und/oder Behandlung funktioneller gastrointestinaler Erkrankungen.

Daher betrifft die vorliegende Erfindung bevorzugt auch die Verwendung der erfindungsgemäßen Derivate zur Herstellung eines Arzneimittels zur Diagnose, Prophylaxe und/oder Behandlung funktioneller gastrointestinaler Erkrankungen.

Insbesondere sind die erfindungsgemäßen Derivate, im Gegensatz zu anderen Verbindungen mit ähnlichem Wirkungsspektrum, besonders geeignet für die Behandlung entzündlicher Darmerkrankungen und zur Verwendung in pharmazeutischen Zubereitungen zur Behandlung entzündlicher Darmerkrankungen, da sie neben der analgetischen und antiinflammatorischen Wirkung geeignet sind, durch die Erkrankung hervorgerufene Störungen der Darmmotorik zu normalisieren. Insbesondere sind sie geeignet, die Darmbewegungen wieder in Gang zu bringen, wenn durch die entzündliche Darmerkrankung ein Darmverschluss droht oder bereits eingetreten ist. Auch kann diese Wirkung zur Behandlung eines postoperativen Ileus und der damit verbundenen Schmerzen eingesetzt werden. Entzündliche Darmerkrankungen führen häufig zu Dickdarmschmerzen, Verdauungsstörungen und im schlimmsten Fall zu einem Darmverschluss. Insbesondere letzterer ist oft mit kolikartigen Schmerzen infolge eines heftigen Kontraktionsreizes, Stuhl - und Windverhalten, Erbrechen und, mit zunehmender Dauer des Zustandes, Dehydratation, Abwehrspannung des Abdomens und schliesslich einem Schock verbunden. Zur Behandlung und Linderung der vorgenannten Erkrankungen bzw. Symptome können die erfindungsgemäßen Derivate vorteilhaft eingesetzt werden. Insbesondere lindern Sie bei entzündlichen Darmerkrankungen die damit verbundenen Schmerzen und können im akuten Fall eines durch die entzündliche Darmerkrankung drohenden bzw. hervorgerufenen Darmverschlusses die Motorik des Darms wieder normalisieren oder wieder in Gang setzen, ohne spürbare Nebenwirkungen hervorzurufen, wie beispielsweise in der EP-A-0 752 246 beschrieben

Daher sind die erfindungsgemäßen Derivate zur Behandlung und/oder Prophylaxe von entzündlichen Darmerkrankungen und insbesondere die Verwendung der erfindungsgemäßen Derivate zur Herstellung eines Arzneimittels zur Behandlung und/oder der Prophylaxe von entzündlichen Darmerkrankungen ein bevorzugter Aspekt der vorliegenden Erfindung.

Die vorliegende Erfindung betrifft auch die Verwendung der erfindungsgemäßen Derivate zur Herstellung eines Arzneimittels zur Diagnose, Prophylaxe und/oder Behandlung der Neuropathie, der damit verbundenen klinischen Bilder und Symptomen sowie der damit verwandten Krankheiten.

Neuropathie, oder periphere Neuropathie, ist ein allgemein bekannter Begriff betreffend Erkrankungen der peripheren Nerven, üblicherweise eine Nervenschädigung.

Das periphere Nervensystem besteht aus Nerven, die aus dem Rückenmark in alle Teile des Körpers verzweigen. Eine Nervenschädigung, die in nur einem Teil des Körpers auftritt, bezeichnet man als Mononeuropathie, eine Nervenschädigung in mehreren Bereichen als Polyneuropathie. Mit Radikulitis bezeichnet man eine Neuropathie, welche die Nervenwurzeln betrifft. Wenn die Krankheit symmetrisch auf beiden Seiten des Körpers auftritt, bezeichnet man den Zustand als symmetrische Neuropathie. Eine periphere Neuropathie kann als Folge von Diabetes, Vitaminmangel, HIV, Krebs, Viruserkrankungen, Alkoholmissbrauch oder als Nebenwirkung von Pharmazeutika auftreten. Sie kann daher auch nach der Ursache kategorisiert werden, wie beispielsweise als diabetische Neuropathie, ernährungsbedingte Neuropathie oder alkohol-induzierte Neuropathie. Wenn eine Ursache nicht bestimmt werden kann, nennt man den Zustand idiopathische Neuropathie. Periphere Neuropathien sind weit verbreitet, stören oft das Allgemeinbefinden des Betroffenen erheblich und führen nicht selten zu Behinderungen. Die Ätiologie, das Krankheitsbild, das Auftreten und/oder die Wechselwirkungen mit anderen Krankheiten sind in der Literatur ausführlich diskutiert worden, z. B. in Boulton, Diabetes Metab 1998; 24 (Suppl 3): 55-65; IIIa, Eur Neurol 1999; 41 (Suppl 1): 3-7; Lagueny, Rev Prat 2000; 50: 731-735; Peltier and Russell, Curr Opin Neurol 2002; 15: 633-638; Simpson, J Neurovirol 2002; 8 (Suppl 2): 33-41; Sweeney, Clin J Oncol Nurs 2002; 6: 163-166; und Wulff und Simpson, Semin Neurol 1999; 19: 157-164).

Die am meisten verbreitete Komplikation des Diabetes ist eine Neuropathie. Es wird geschätzt, dass bis zu 60 Prozent der Diabetes-Patienten als Folge des Diabetes eine Neuropathie entwickeln. Die Neuropathie ist typischerweise mit einer breiten Vielfalt an Symptomen verbunden, unter anderem Taubheitsgefühlen oder Kribbeln, sehr unangenehmen Stichen (pins and needles sensation), dem Gefühl, man bekäme eine Serie von elektrischen Reizen, und Schmerzen verschiedener Art und Stärke. Diese Symptome können einzeln oder in Kombination auftreten.

Die Symptome der diabetischen Neuropathie und insbesondere der damit verbundene Schmerz betrifft meistens die Füße und die Knöchel sowie zu einem geringeren Anteil die Beine oberhalb der Knie und die Arme. Eine unzureichende Einstellung des Blutzuckerspiegels führt regelmäßig zu einer Nervenschädigung, welche wiederum mit ziemlicher Sicherheit zur Entwicklung des Krankheitsbilds einer diabetischen Neuropathie führt. Für die diabetische Neuropathie ist der Auslöser bekannt, nämlich Diabetes mellitus, und man geht davon aus, dass er mit einer unzureichenden Einstellung des Blutzuckerspiegels und der damit einhergehenden Hyperglykämie zusammenhängt. Je höher der Blutzuckerspiegel ist und je länger er oberhalb der Norm bleibt, umso schwerer wird im Regelfall die Krankheit sein. Der exakte Mechanismus, wie erhöhte Blutzuckerwerte zu einer Nervenschädigung führen , ist noch zu erforschen; andere Faktoren, wie beispielsweise Anomalien der Nerven-Wachstumsfaktoren oder der Einfluss von kardiovaskulären Erkrankungen (Ischämien, Hypoxien) sind ebenfalls als wichtige, zur Entwicklung einer diabetischen Neuropathie beitragende Faktoren postuliert worden (siehe beispielsweise Jude und Boulton, Diabetes Reviews 1999; 7: 395-410; Dworkin, Clin J Pain 2002; 18: 343-349; Simmons and Feldman, Curr Opin Neurol 2002; 15: 595-603; Barbano et al., Curr Pain Headache Rep 2003; 7: 169-177; Spruce et al., Diabet Med 2003; 20: 88-98).

Überraschenderweise wurde gefunden, dass die erfindungsgemäßen Derivate erfolgreich in der Diagnose, Prophylaxe und/oder Behandlung von Neuropathie, den damit verbundenen Krankheitsbildern und Symptomen sowie verwandten Krankheiten eingesetzt werden kann.

Darüber hinaus beschleunigen die erfindungsgemäßen Derivate wie hierin beschrieben vorzugsweise die Nervenregeneration und beschleunigen oder induzieren daher besonders bevorzugt die Heilung der hierin beschriebenen pathologischen Zustände oder Krankheiten, wie beispielsweise eine teilweise oder vollständige Ausheilung der Neuropathie. Darüber hinaus zeigen die erfindungsgemäßen Derivate wie hierin beschrieben vorzugsweise weniger Nebenwirkungen als die Pharmazeutika des Standes der Technik.

Daher ist die Verwendung der erfindungsgemäßen Derivate zur Herstellung eines Arzneimittels zur Diagnose, Prophylaxe und/oder Behandlung von Neuropathien, der damit verbundenen Krankheitsbilder und Symptome sowie verwandten Krankheiten ebenfalls bevorzugter Gegenstand der vorliegenden Erfindung.

Im Sinne der Erfindung ist die Neuropathie vorzugsweise ausgewählt unter Diabetes-induzierter Neuropathie, ernährungsbedingter Neuropathie, Vitaminmangel-induzierter Neuropathie, HIV-induzierter Neuropathie, Krebs-induzierter Neuropathie, Virus-induzierten Neuropathien, Alkoholmissbrauch-induzierter Neuropathie und Arzneimittel-induzierter Neuropathie, besonders bevorzugt Diabetes-induzierter Neuropathie, ernährungsbedingter Neuropathie und Alkoholmissbrauch-induzierter Neuropathie, und insbesondere Diabetes-induzierten Neuropathien.

Die erfindungsgemäßen Derivate zeigen außerdem eine hohe Wirksamkeit bei Neuropathien anderer Ätiologie, und verwandten Erkrankungen, Krankheitsbildern oder Indikationen, wie beispielsweise Neuralgien nach Herpes-Infektionen, Chemotherapie-induzierte Neuropathie, Vulvovaginitis; und/oder Lupus erythematodes. Die Aktivität oder Wirksamkeit der erfindungsgemäßen Derivate in der Prophylaxe und/oder Behandlung der genannten Erkrankungen kann nach aus dem Stand der Technik bekannten Methoden oder in Analogie dazu gezeigt werden, beispielsweise wie beschrieben in Backonja und Glanzman, Clin Ther 2003; 25: 81-104; Bates und Timmins, Int J STD AIDS 2002; 13: 210-212; Carrazana und Mikoshiba, J Pain Symptom Manage 2003; 25(5 Suppl): S31-35; Harel et al., Pediatr Neurol 2002; 27: 53-56; Jensen, Eur J Pain 2002; 6 (Suppl A): 61-68; LaSpina et al. Eur J Neurol 2001; 8: 71-75; Lersch et al., Clin Colorectal Cancer 2002; 2: 54-58; und/oder Mellegers et al., Clin J Pain 2001; 17: 284-295), oder in analoger Weise dazu.

Die Aktivität oder Wirksamkeit der erfindungsgemäßen Derivate kann nach aus dem Stand der Technik bekannten Verfahren oder Methoden, oder in analoger Weise dazu, ermittelt werden. Geeignete Verfahren umfassen experimentelle nicht-klinische Verfahren, wie beispielsweise in vitro Assays, in vivo Assays, zelluläre Assays und Tiermodelle, und klinische Methoden oder klinische Studien, sind aber nicht beschränkt darauf. Geeignete Verfahren sind beispielsweise beschrieben in in Field et al., Pain 1999; 80: 391-398; Miki et al., Eur J Pharmacol 2001; 430: 229-234; Wallin et al., Eur J Pain 2002; 6: 261-272); Backonja, Epilepsia 1999; 40 (suppl 6): S57-59; Gorson et al., J Neurol Neurosurg Psychiatry 1999; 66: 251-252; Dallocchio et al., J Pain Symptom Manage 2000; 20: 280-285; Hemstreet und Lapointe, Clin Ther 2001; 23: 520-531; Brooks-Rock, Nurse Pract 2001; 26: 59-61; Backonja und Glanzman, Clin Ther 2003; 25: 81-104; Kaul et al., Arch Int Pharmacodyn Ther 1978; 234: 139-44; und Calcutt et al., Anesthesiology 2000; 93:1271-1278).

Zum Beispiel wird der Streptozotozin-induzierte Diabetes an der Ratte als geeignetes Tiermodell für das Studium des Diabetes Typ 1 (Insulinabhängiger Diabetes) und/oder der Folgeerkrankungen und der dazugehörenden Symptome, insbesondere der hierin beschriebenen Folgeerkrankungen und der dazugehörenden Symptome, angesehen (siehe beispielsweise: Kaul et al., Arch Int Pharmacodyn Ther 1978; 234: 139-44; Calcutt et al., Anesthesiology 2000; 93:1271-1278). In diesem Modell führt bereits der Streptozotin-induzierte Kurzzeit-Diabetes bei Ratten zu sensorischen Störungen, von thermischer Hypoalgesie bis zu übersteigerten Verhaltensantworten auf andere sensorische Stimuli. Eine Fehlemährung kann die sensorischen Nervenstörungen während des Diabetes verstärken.

Im Zusammenhang mit der Diagnose, Prophylaxe und/oder Behandlung der Neuropathie, der damit verbundenen klinischen Bilder und Symptome sowie der damit verwandten Krankheiten und insbesondere der Verwendung der erfindungsgemäßen Derivate zur Herstellung eines Arzneimittels zur Anwendung in der Diagnose, Prophylaxe und/oder Behandlung der Neuropathie, der damit verbundenen klinischen Bilder und Symptome sowie der damit verwandten Krankheiten wird auf die europäische Patentanmeldung des gleichen Anmelders vom 30.10.2003 mit der Europäischen Anmeldenummer EP 03024781.1 und deren Internationale bzw. Europäische Folgeanmeldungen verwiesen.

Ferner eignen sich die erfindungsgemäßen Derivate zur Behandlung und/oder Prophylaxe von Schmerzen und Schmerzüberempfindlichkeiten, insbesondere solchen, die bei Rückenleiden, Brandverletzungen, Sonnenbrand und rheumatischen Erkrankungen auftreten, sowie den dabei auftretenden entzündlichen Reaktionen. Insbesondere lassen sich bei diesen Indikationen durch die Verabreichung von geeigneten pharmazeutischen Zubereitungen, die die erfindungsgemäßen Derivate enthalten, neben den eigentlichen Schmerzen und Schmerzüberempfindlichkeitsreaktionen entzündliche Vorgänge mit beeinflussen. Auch lässt sich der bei schwersten Verbrennungen auftretende reflektorische Ileus verhindern bzw. behandeln. Die Verwendung der erfindungsgemäßen Derivate zur Herstellung eines Arzneimittels zur Behandlung und/oder Prophylaxe der vorstehend genannten Erkrankungen und/oder Symptome ist ebenfalls Gegenstand der vorliegenden Erfindung.

Ferner wurde gefunden, dass die erfindungsgemäßen Derivate eine vorteilhafte Wirkung bei der Behandlung von Allergien, insbesondere Sonnenallergien hinweisen, da unter Einfluss der erfindungsgemäßen Derivate allergische Hautreaktionen schnell abklingen und der damit verbundene Juckreiz schnell nachlässt. Gleichermaßen wurden positive Ergebnisse bei der Behandlung von Neurodermitis und insbesondere Pruritus (Juckreiz, englisch: Itching) beobachtet. Insbesondere lässt bei diesen Erkrankungen unter Einwirkung der erfindungsgemäßen Derivate der Juckreiz der Haut nach und durch die Erkrankung auftretende oder geförderte entzündliche Reaktionen der Haut werden günstig beeinflusst. Die Verwendung der erfindungsgemäßen Derivate zur Herstellung eines Arzneimittels zur Behandlung und/oder Prophylaxe der vorstehend genannten Erkrankungen und/oder Symptome ist ebenfalls Gegenstand der vorliegenden Erfindung. Besonders bevorzugt ist daher die Verwendung der erfindungsgemäßen Derivate zur Prophylaxe und/oder Behandlung von Neurodermitis, Juckreiz und insbesondere Pruritus, und insbesondere die Verwendung zur Herstellung eines Arzneimittels zur Behandlung und/oder Prophylaxe von Neurodermitis, Juckreiz und insbesondere Pruritus. Auch in diesem Zusammenhang wird auf die Offenbarung der EP-A 0 752 246 ausdrücklich Bezug genommen.

Ferner eignen sich die erfindungsgemäßen Derivate zur Behandlung von postoperativen Schmerzen und Schmerzüberempfindlichkeitsreaktionen, sowie des häufig nach Abdominaloperationen auftretenden Ileus. Die erfindungsgemäßen Derivate zur Behandlung und/oder zur Herstellung eines Arzneimittels zur Behandlung der vorstehend genannten Erkrankungen und/oder Symptome sind ebenfalls Gegenstand der vorliegenden Erfindung.

Die erfindungsgemäßen Derivate sind ferner zur Behandlung und/oder Prophylaxe von nicht entzündlichen Erkrankungen des Gastrointestinaltraktes, bevorzugt nicht-entzündlichen Darmerkrankungen und insbesondere zur Behandlung und/oder Prophylaxe des Reizdarms, auch Irritable Bowel Syndrom (IBS) genannt, einsetzbar und wirksam, da sie die gleichzeitig die mit dieser Erkrankung verbundenen Schmerzen lindern und die Erkrankung heilen können. Vorteilhaft ist dabei, dass die erfindungsgemäßen Derivate auf eine normale Darmperistaltik keine Auswirkungen haben, jedoch die Ausheilung des Irritable Bowel Syndroms mitbewirken. Somit können die erfindungsgemäßen Derivate vorteilhaft zur Prophylaxe von Reizdarm eingesetzt werden. Die Erfindungsgemäßen Derivate und insbesondere die Derivate der Formel I sind im Gegensatz zu anderen Verbindungen mit ähnlichem Wirkungsspektrum besonders geeignet für die Verwendung in pharmazeutischen Zubereitungen zur Behandlung des Reizdarms, da sie neben der analgetischen und antiinflammatorischen Wirkung geeignet sind, durch die Erkrankung hervorgerufene Störungen der Darmmotorik zu normalisieren. In diesem Zusammenhang wird auf die DE 198 49 650 verwiesen. Die Wirksamkeit der erfindungsgemäßen Derivate in diesen Indikationen kann nach aus dem Stand der Technik bekannten Methoden nachgewiesen werden, z. B. wie in Delgado-Aros et al., Am. J. Physiol. Gastrointest Liver Physiol. 284: G558-G566, 2003, oder in Analogie dazu.

Daher sind die erfindungsgemäßen Derivate zur Behandlung und/oder Prophylaxe von nicht entzündlichen Erkrankungen des Gastrointestinaltraktes, bevorzugt nicht-entzündlichen Darmerkrankungen und besonders bevorzugt Reizdarm, ein bevorzugter Aspekt der vorliegenden Erfindung. Die Verwendung der erfindungsgemäßen Derivate zur Herstellung eines Arzneimittels zur Behandlung und/oder Prophylaxe von nicht entzündlichen Erkrankungen des Gastrointestinaltraktes, bevorzugt nicht-entzündlichen Darmerkrankungen und besonders bevorzugt Reizdarm, ist daher ein besonders bevorzugter Aspekt der vorliegenden Erfindung.

Die erfindungsgemäßen Derivate zeigen darüber hinaus bei Verabreichung an Patienten wenigstens eine der folgenden vorteilhaften Eigenschaften:
- die erfindungsgemäßen Derivate sind effektiv als Modulatoren des Tonus des Gastrointestinaltrakts; insbesondere sind sie geeignet, um eine Relaxation oder Aktivierung des Tonus des Gastrointestinaltrakts zu bewirken; im allgemeinen ist die modulierende Wirkung der erfindungsgemäßen Derivate dosisabhängig;
- die erfindungsgemäßen Derivate sind geeignet, das Sattheitsgefühl und/oder die so genannten postprandialen Symptome, z. B. die Menge und/oder Stärke der Blähungen, das Völlegefühl, die Übelkeit und/oder die Schmerzen nach der Nahrungsaufnahme, zu beeinflussen;
- die Auswirkungen der erfindungsgemäßen Derivate auf die Sattheit und/oder die postprandialen Symptome ist vorzugsweise Dosisabhängig; im allgemeinen führen geringe Dosen zu einer Abnahme der Symptome, während höhere Dosen zu einer Zunahme der Symptome führen können;
- die erfindungsgemäßen Derivate können das Volumen, insbesondere das Aufnahmevolumen, und/oder die Verträglichkeit, z. B. gegenüber mechanischen Reizen, des Gastrointestinaltraktes und insbesondere des Colons beeinflussen; beispielsweise kann das Volumen durch die Verabreichung niedrigerer bis mittlerer Dosen deutlich gesteigert werden im Vergleich zu dem Zustand ohne Verabreichung eines erfindungsgemäßen Derivats;
- im allgemeinen zeigt die Verabreichung der erfindungsgemäßen Derivate keine oder geringe negative Auswirkungen auf funktionale Parameter des Gastrointestinaltrakts, wie beispielsweise sie Durchgangszeit durch den Gastrointestinaltrakt, die gastrische Entleerung, die intestinale und die colonische Entleerung; dieser Effekt ist vorzugsweise nicht oder nur wenig Dosisabhängig; somit zeigt die Verabreichung der erfindungsgemäßen Derivate geringe Auswirkungen auf die natürliche Funktion des Gastrointestinal Trakts und somit eine geringe Tendenz zu unerwünschten Nebenwirkungen;
- vorzugsweise führt die Verabreichung der erfindungsgemäßen Derivate in höheren Dosen zu einer Zunahme/Verstärkung der Symptome betreffend das Völlegefühl; beispielsweise haben Patienten früher das Gefühl der Magenvölle, obwohl sie wenig Nahrung zu sich genommen haben und essen daher weniger; somit können die erfindungsgemäßen Derivate zur Korrektur des fehlenden Völlegefühls bei Obese-Patienten und daher zur Behandlung von Obesität eingesetzt werden;
- Die erfindungsgemäßen Derivate sind ferner zur Behandlung und/oder Prophylaxe von Essstörungen und/oder Verdauungsstörungen, insbesondere psychogenen Essstörungen und/oder psychogenen Verdauungsstörungen, einsetzbar und wirksam, da sie geeignet sind, den Tonus des Gastrointestinaltraktes vorteilhaft zu beeinflussen oder zu modulieren.

Die erfindungsgemäßen Derivate eignen sich daher zur Behandlung und/oder Prophylaxe von Ess- und Verdauungsstörungen, insbesondere psychogenen Ess- und Verdauungsstörungen, wie pathologisch veränderter Appetit, insbesondere Appetitverlust oder verringerter Appetit, wie er beispielsweise bei der Schwangerschaft, bei Krebs, bei Infektionskrankheiten, z. B. Grippe oder HIV, als postoperative Nebenwirkung, als Folge von Katabolismus, Kachexie, Anorexie, insbesondere Anorexia nervosa, Dysorexie, Dysponderosis, Adipositas Polyamin, Obesität, Gastroparese, insbesondere neurogene Gastroparese, diabetische Gastroparese, myogen der Gastroparese oder durch Drogen induzierte Gastroparese, Gastroatonie, Gastroparese Lösungen oder Enteroparese, insbesondere nach gastrointestinalen Operationen, und Stenosen des Gastrointestinaltrakts, insbesondere Stenosen des Pylorus.

Ferner eignen sich die erfindungsgemäßen Derivate zur Verabreichung als Appetitzügler, einzelnen oder in Kombination mit anderen Appetitzüglern, vorzugsweise mit einem oder mehreren Sympathomimetika. Geeignete weitere Appetitzügler oder Sympathomimetika sind dem Fachmann bekannt. Geeignet als Appetitzügler oder Sympathomimetika sind insbesondere Phenylpropanolamin, Cathin, Sibutramin, Amfepramon, Ephedrin und Norpseudoephedrin. In diesem Zusammenhang wird auf die EP 0 201 1047.4 verwiesen, auf deren Offenbarung hiermit in vollem Umfang Bezug genommen wird.

Gegenstand der vorliegenden Erfindung ist daher die Verwendung der erfindungsgemäßen Derivate zur Herstellung eines Arzneimittels zur Verabreichung zusammen mit den erfindungsgemäßen Derivaten verschiedenen Appetitzüglern. Gegenstand der vorliegenden Erfindung ist daher auch die Verwendung der erfindungsgemäßen Derivate zur Herstellung eines Kombinationsarzneimittels, enthaltend wenigstens einen weiteren Appetitzügler.

Die Dosisabhängigkeit der vorteilhaften Effekte der erfindungsgemäßen Derivate auf den Gastrointestinaltrakt kann nach üblichen Methoden einfach bestimmt werden, beispielsweise wie in der EP 02011047.4 beschrieben, oder in Analogie dazu. Niedrigere Dosen (in mg Wirkstoff, berechnet als Asimadolin, pro kg Körpergewicht) im Sinne dieser Erfindung liegen vorzugsweise im Bereich von 0.001 bis 0.5 mg/kg täglich, besonders bevorzugt von 0.01 bis 1.0 mg/kg täglich und insbesondere von 0.1 bis 2.0 mg/kg täglich, beispielsweise bei etwa 0.3 mg/kg täglich, etwa 0.75 mg/kg täglich oder etwa 1.0 mg/kg täglich, wohingegen höhere Dosen im Sinne der Erfindung in der Regel oberhalb von 2.0 mg/kg täglich und vorzugsweise im Bereich von 2.25 bis 5mg/kg täglich und insbesondere im Bereich von 2.5 mg/kg bis 10 mg/kg täglich liegen, z. B. bei etwa 3 mg/kg täglich, etwa 5 mg/kg täglich oder etwa 8 mg/kg täglich.

Darüber hinaus gibt es Hinweise darauf, dass die erfindungsgemäßen Derivate vorteilhaft zur Behandlung von Augenschmerzen, insbesondere zur topischen Behandlung von Augenschmerzen, eingesetzt werden kann, unabhängig von der Genese der Augenschmerzen. Besonders vorteilhaft können die erfindungsgemäßen Derivate zur topischen Behandlung von postoperativen Augenschmerzen eingesetzt werden, wie sie beispielsweise nach Operationen mittels Lasern und insbesondere nach so genannten PRK-Operationen auftreten können. Dabei steht PRK für fotorefraktive Keratotomie.

Ferner gibt es Hinweise darauf, dass die erfindungsgemäßen Derivate vorteilhaft zur Behandlung von Ohrenschmerzen, insbesondere zur topischen oder intranasalen Behandlung von Ohrenschmerzen, eingesetzt werden kann, unabhängig von der Genese der Ohrenschmerzen. Besonders vorteilhaft können die erfindungsgemäßen Derivate zur Behandlung von Ohrenschmerzen eingesetzt werden, wie sie beispielsweise bei Otitis, Infektionen, Entzündungen, insbesondere Mittelohrentzündung, Ödemen, Unfalltraumata, Operationen und postoperativ auftreten können.

Vorzugsweise können die erfindungsgemäßen Derivate vorteilhaft zur Behandlung und/oder Prophylaxe von Dyspepsien, insbesondere von nicht mit einem Ulcus verbundenen Dyspepsien (Non-Ulcer Dyspepsia oder NUD), eingesetzt werden.

Vorzugsweise können die erfindungsgemäßen Derivate ferner vorteilhaft zur Behandlung und/oder Prophylaxe von Neuropathien, insbesondere diabetischen Neuropathien (Diabetic Neuropathy), eingesetzt werden.

Krankheiten oder Indikationen im Sinne der Erfindung sind daher vorzugsweise ausgewählt unter Schmerzen, Schmerzzuständen, Ohrenschmerzen, Augenschmerzen, Entzündungen, Ileus, entzündlichen Darmerkrankungen, Reizdarm, Reizblase, Dyspepsie, Neuropathie, Adipositas, Bulimie, Fettsucht, Kachexie, Anorexie, Dysorexie, Dysponderosis, Gastroparese und Stenosen des Gastrointestinaltraktes.

Gegenstand der vorliegende Erfindung sind daher die erfindungsgemäßen Derivate und/oder ein Salz oder Solvat davon, zur Prävention und/oder Behandlung von Krankheiten, insbesondere einer oder mehrerer der hierin genannten Krankheiten oder Indikationen.

Bevorzugter Gegenstand der vorliegenden Erfindung ist daher die Verwendung eines erfindungsgemäßen Derivats und/oder eines Salzes oder Solvates davon, zur Herstellung eines Arzneimittels zur Prävention und/oder Bekämpfung von Krankheiten, insbesondere einer oder mehrerer der hierin genannten Krankheiten oder Indikationen.

Als besonders vorteilhaft hat sich außserdem bei den erfindungsgemäßen Derivaten erwiesen, dass sie trotz ihres wie vorstehend beschrieben vorteilhaft modifizierten Eigenschaftsprofls offenbar die Blut-Him-Schranke nicht passieren können und daher kein Abhängigkeitspotential aufweisen. Auch wurden bisher keine Wirkungen gefunden, die die Nutzung der vorteilhaften Wirkungen für die beanspruchten Indikationen in irgendeiner Weise einschränken würden. Dies ist insbesondere deshalb überraschend, da die erfindungsgemäßen Derivate in der Regel eine höhere, bevorzugt sogar eine stark erhöhte Polarität und/oder Hydrophilie aufweisen, welche die Fähigkeit zur Überwindung der Blut-Hirn-Schranke regelmäßig stark erhöht.

Gegenstand der vorliegenden Erfindung ist daher die Verwendung der erfindungsgemäßen Derivate zur Herstellung eines Arzneimittels zur Behandlung und/oder Prophylaxe der vorstehend genannten Krankheiten.

Gegenstand der vorliegenden Erfindung ist daher auch eine pharmazeutische Zubereitung, die wenigstens eins der erfindungsgemäßen Derivate und/oder ein Solvat oder Salz davon enthält. Vorzugsweise enthalten die erfindungsgemäßen pharmazeutischen Zubereitungen, bezogen auf das Gesamtgewicht der pharmazeutischen Zubereitung, wenigstens 0,001 Gew.-%, besonders bevorzugt wenigstens 0,01 Gew.-%, ganz besonders bevorzugt wenigstens 0,1 Gew.-% und insbesondere wenigstens 1,0 Gew.-% wenigstens eines erfindungsgemäßen Derivats und/oder eines Solvates oder Salzes davon. In der Regel enthalten die erfindungsgemäßen Zubereitungen höchstens 100 Gew.-% und vorzugsweise höchstens 98 Gew.-% oder höchstens 95 Gew.-% an erfindungsgemäßen Derivaten. In der Regel enthalten die erfindungsgemäßen pharmazeutischen Zubereitungen wenigstens 5 Gew.-%, beispielsweise wenigstens 15 Gew.-% oder wenigstens 30 Gew.-% weitere, von den erfindungsgemäßen Derivaten und den Salzen und Solvaten davon verschiedene Komponenten, vorzugsweise ausgewählt unter weiteren Wirkstoffen und den von Wirkstoffen verschiedenen üblichen Bestandteilen, die pharmazeutische Zubereitungen in der Regel umfassen. Beispiele für solche weiteren, von Wirkstoffen verschiedenen Komponenten sind hierin beschrieben. Beispiele für solche weitere, von den erfindungsgemäßen Derivaten und den Salzen und Solvaten davon verschiedene Wirkstoffe sind hierin beschrieben.

Die erfindungsgemäßen Derivate werden vorzugsweise ganz oder teilweise nach konventionellen chemischen Syntheseverfahren, biotechnologischen Verfahren oder gentechnischen Verfahren hergestellt. Bei der Herstellung der erfindungsgemäßen Derivate wird vorzugsweise wenigstens die kovalente Bindung zwischen dem N-methyl-N-[(1S)-1-phenyl-2-((3S)-3-hydroxypyrrolidin-1-yl)ethyl]-2,2-diphenylacetamid und der kovalent gebundenen Säure mittels konventioneller chemischer Syntheseverfahren durchgeführt. Ein geeignetes konventionelles chemisches Syntheseverfahren wird nachstehend beschrieben.

Gegenstand der Erfindung ist ferner ein Verfahren zur Herstellung der erfindungsgemäßen Derivate sowie ihrer physiologisch unbedenklichen Salze, dadurch gekennzeichnet, dass man
a) eine Verbindung der Formel II 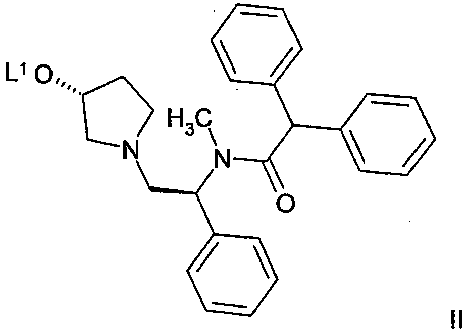 worin
   - L¹: H oder ein Metallion bedeutet;
b) mit einer Verbindung der Formel III,

   R¹-L² III

   worin
   - L²: für eine Abgangsgruppe, vorzugsweise ausgewählt unter Cl, Br, I, OH, SR³, einer reaktionsfähig veresterten OH-Gruppe, einer Imidazolid-Gruppe, einer Carboxylat-Gruppe und einer Diazoniumgruppe, steht, R³ Alkyl, Aralkyl oder Aryl bedeutet, und R¹ die vor- und nachstehend für die Verbindungen der Formel I angegebene Bedeutungen hat, oder, wenn R¹ zusätzlich zu der Gruppe L² eine oder mehrere funktionelle Gruppen, vorzugsweise eine oder mehrere funktionelle Gruppen, ausgewählt unter Hydroxygruppen und Carboxylgruppen, aufweist, ein ganz oder teilweise mit Schutzgruppen versehenes Derivat von R¹ umsetzt,
c) gegebenenfalls eine oder mehrere Schutzgruppen von R¹ abspaltet, sofern das Produkt der Umsetzung von a) und b) eine oder mehrere der Schutzgruppen umfasst; gegebenenfalls die Verbindung der Formel I isoliert,
d) die erhaltene Verbindung der Formel I durch Behandeln mit einer Säure oder einer Base in eines ihrer Salze überführt und gegebenenfalls das Salz isoliert.

In den Verbindungen der Formel II bedeutet L¹ vorzugsweise H oder ein Metallion. Geeignete Metallionen sind insbesondere Alkalimetall-, Erdalkalimetall- oder Aluminium-Ionen. Bevorzugt als Metallionen sind Alkalimetallionen, insbesondere Li, Na oder K. Besonders bevorzugt steht L¹ für H.

In den Verbindungen der Formel III ist R¹ vorzugsweise ausgewählt unter
a) Acylresten wie vorstehend für die Verbindungen der Formel I beschrieben
b) Acylresten, die eine Hydroxy-Gruppe und/oder einen oder mehrere Carboxylgruppen aufweisen wie vorstehend für die Verbindungen der Formel I beschrieben,
c) Alkylresten, die wie vorstehend für die Verbindungen der Formel I beschrieben von Polyhydroxymonocarbonsäuren abgeleitet sind, und
d) wie vorstehend für die Verbindungen der Formel I beschriebenen Sulfonsäuregruppen, Phosphonsäuregruppen und Nitrogruppen.

In den Verbindungen der Formel III steht L² für eine geeignete Abgangsgruppe. Geeignete Abgangsgruppen sind dem Fachmann bekannt, z. B. aus Houben-Weyl, Methoden der organischen Chemie, Thieme-Verlag, Stuttgart. Beispiele für geeignete Abgangsgruppen sind neben den Halogeniden F, Cl, Br und I und reaktionsfähig veresterten OH-Gruppen vor allen Dingen Imidazolide, von Säureanhydriden abgeleitete Säure-Anionen, wie sie beispielsweise entstehen, wenn als Verbindung der Formel III ein gemischtes bzw. unsymmetrisches oder ein symmetrisches Säureanhydrid eingesetzt wird, und Diazonium-Gruppen, wie sie beispielsweise durch Diazotierung von Aminen nach üblichen Methoden erhalten werden können.

Reaktionsfähig veresterte OH-Gruppen im Sinne der Erfindung sind vorzugsweise Alkylsulfonyloxy-Gruppen mit 1-6 C-Atomen (bevorzugt Methylsulfonyloxy) oder Arylsulfonyloxy-Gruppen mit 6-10 C-Atomen (bevorzugt Phenyl- oder p-Tolylsulfonyloxy).

In vielen Fällen setzt man im erfindungsgemäßen Verfahren vorteilhaft als Verbindung der Formel III ein Säurehalogenid ein, vorzugsweise ein Säurehalogenid, dass von vorstehend genannten Säuren abgeleitet ist. Geeignete Säurehalogenide der vorstehend genannten Säuren und Verfahren zu ihrer Herstellung sind dem Fachmann bekannt. Bevorzugt als Säurehalogenid ist Chlorsulfonsäure.

In vielen Fällen setzt man im erfindungsgemäßen Verfahren vorteilhaft als Verbindung der Formel III ein Säureanhydrid ein, vorzugsweise ein Säureanhydrid, das von vorstehend genannten Säuren abgeleitet ist bzw. wenigstens eine der vorstehend/nachstehend zur Herstellung der erfindungsgemäßen Derivate genannten Säuren enthält. Geeignete Säureanhydride zur Herstellung der erfindungsgemäßen Derivate nach dem erfindungsgemäßen Verfahren sind dem Fachmann bekannt. Bevorzugt als Säureanhydrid ist Essigsäureanhydrid.

In den Verbindungen der Formel III ist L² vorzugsweise ausgewählt unter Cl, Br und SR³ und ist insbesondere Br.

In den Verbindungen der Formel III ist L² vorzugsweise von OH und/oder von Diazonium-Gruppen verschieden.

In den Verbindungen der Formel III, in denen L² für SR³ steht, ist R³ vorzugsweise ausgewählt unter verzweigten oder unverzweigten Alkylresten mit 1 bis 10 Kohlenstoffatomen, wie vorzugsweise Methyl, Ethyl, n-Proply, Isopropyl, n-Butyl, Isobutyl, tert.-Butyl, insbesondere Ethyl, Aralkylresten mit 6 bis 10 Kohlenstoffatomen, wie Benzyl, und Arylresten mit 6 bis 10 Kohlenstoffatomen, wie Phenyl, p-Nitrophenyl und p-Toluolyl. Besonders bevorzugt sind Methyl, Ethyl, Isopropyl, n-Butyl, Isobutyl, Phenyl und Benzyl. Bevorzugte Gruppen SR³ sind ausgewählt unter S-C₂H₅, S-CH(CH₃)₂, S-C₆H₅ und S-CH₂-C₆H₅.

Das erfindungsgemäße Verfahren kann in Abwesenheit oder Anwesenheit, vorzugsweise in Anwesenheit eines geeigneten, unter den Reaktionsbedingungen vorzugsweise inerten Lösungsmittels durchgeführt werden. Geeignete Lösungsmittel sind dem Fachmann bekannt. Bevorzugte Lösungsmittel, insbesondere für die Reaktionsschritte a) und b), sind polare, nicht protische Lösungsmittel, wie Acetonitril, Tetrahydrofuran (THF), 1,4-Dioxan und Dichlormethan. Besonders geeignete Lösungsmittel für die Reaktionsschritte c) und/oder d) sind die Lösungsmittel, die zur Entfernung der jeweiligen Schutzgruppe vorgeschlagen sind.

Die Reaktionszeiten für das erfindungsgemäße Verfahren liegen in der Regel zwischen einigen Minuten und wenigen Tagen, vorzugsweise zwischen 30 Minuten und 48 Stunden und insbesondere zwischen einer Stunde und 24 Stunden.

Die Reaktionstemperaturen für das erfindungsgemäße Verfahren liegen in der Regel zwischen - 20 °C und 100 °C, vorzugsweise zwischen - 10 °C und 60 °C, besonders bevorzugt zwischen 0 °C und 40 °C, z. B. etwa bei Raumtemperatur (25 °C).

Das erfindungsgemäße Verfahren kann in Anwesenheit von Hilfsstoffen durchgeführt werden, welche die Reaktionsgeschwindigkeit, die Selektivität und/oder die Ausbeute des erfindungsgemäßen Verfahrens positiv beeinflussen. Beispiele für solche Hilfsstoffe sind Hilfsbasen, Katalysatoren und Stoffe, die wenigstens eines der entstehenden Produkte und oder Nebenprodukte aus dem Verfahren entfernen. Geeignete solche Hilfsstoffe sind dem Fachmann bekannt, z. B. aus Houben-Weyl, Methoden der organischen Chemie.

Wenn eine Verbindung der Formel III eingesetzt wird, in der L² für Halogen und insbesondere für Chlor oder Brom steht, kann es vorteilhaft sein, bei der Durchführung des erfindungsgemäßen Verfahrens ein Silbersalz zuzusetzen, welches das bei der erfindungsgemäßen Umsetzung der Verbindung der Formel II mit einer Verbindung der Formel III freiwerdende Halogenid als schwer löslichen Niederschlag aus dem Verfahren entfernt. Geeignete Silbersalze sind dem Fachmann bekannt, beispielsweise Silbernitrat, Silbercarbonat, Silberperchlorat. Bevorzugt als Silbersalz ist Silberperchlorat.

Wenn eine Verbindung der Formel III eingesetzt wird, in der L² für SR³ steht, kann es vorteilhaft sein, bei der Durchführung des erfindungsgemäßen Verfahrens ein N-Halogensuccinimid, insbesondere N-lodsuccinimid, und/oder eine Halogencarbonsäure, insbesondere Trifluoressigsäure zuzusetzen.

Wenn der Rest R¹ der Verbindung der Formel III (außer der Gruppe L²) weitere funktionelle Gruppen, insbesondere weitere Hydroxygruppen und/oder Säuregruppen aufweist, ist es in vielen Fällen zweckmäßig, ein so genanntes geschütztes Derivat, d. h. ein mit einer oder mehreren Schutzgruppen versehenes Derivat, eines wie vorstehend beschriebenen Restes R¹ in dem erfindungsgemäßen Verfahren einzusetzen. Dabei werden die weiteren funktionellen Gruppen zweckmäßiger Weise mit den für die jeweilige funktionelle Gruppe üblichen Schutzgruppen geschützt. Geeignete Schutzgruppen und Verfahren zur Herstellung solcher geschützten Derivate sind dem Fachmann bekannt. Hydroxygruppen werden vorzugsweise mit so genannten Hydroxyschutzgruppen geschützt, Säuregruppen vorzugsweise mit so genannten Säureschutzgruppen.

Der Ausdruck "Hydroxyschutzgruppe" ist allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie in der Regel nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl, tert.-Butyl und Acetyl, wobei Acetyl, Benzyl und tert.-Butyl und insbesondere Acetyl besonders bevorzugt sind. Somit liegen geschützte Hydroxygruppen in der Regel in Form von Ethergruppen und/oder Estergruppen vor.

Der Ausdruck "Säureschutzgruppe" ist ebenfalls allgemein bekannt und bezieht sich auf Gruppen, die geeignet sind, eine Säuregruppe, vorzugsweise eine Carbonsäuregruppe oder eine Säuregruppe einer anorganischen Sauerstoffsäure, insbesondere den Säuregruppen vorstehend genannter Säuren vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind unsubstituierte oder substituierte, vorzugsweise unsubstituierte Aryl-, Aralkyl- oder Alkylgruppen. Somit liegen geschützte Säuregruppen vorzugsweise in Form der Aryl-, Aralkyl - oder Alkylester, besonders bevorzugt Aralkyl oder Alkylester, der genannten Säuregruppen vor. Bevorzugt als Säureschutzgruppe sind die methylgruppe, die tert-Butylgruppe und die Benzylgruppe, besonders bevorzugt ist die Methylgruppe. Ester können z.B. mit Essigsäure oder mit NaOH oder KOH in Wasser, Wasser-THF oder Wasser-Dioxan bei Temperaturen zwischen 0 und 100° verseift werden.

Das In-Freiheit-Setzen der geschützten erfindungsgemäßen Derivate und insbesondere geschützten Verbindungen der Formel I gelingt - je nach der benutzten Schutzgruppe - entweder mit Säuren, vorzugsweise starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure, oder Basen, vorzugsweise starken Basen, wie Aminen, Alkalimetallhydroxiden, Erdalkalimetallhydroxiden, Alkalimetallcarbonaten und Erdalkalimetallcarbonaten, vorzugsweise Natrium- oder Kaliumhydroxid, Natrium- oder Kaliumcarbonat. Bevorzugt als Base ist Natriumhydroxid, z. B. eine wässrige Natriumhydroxidlösung. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30°, z. B. bei Raumtemperaturden.

Ester und/oder Ether können beispielsweise vorteilhaft mit Essigsäure oder insbesondere mit NaOH oder KOH in Wasser, Wasser-THF oder Wasser-Dioxan bei Temperaturen zwischen 0 und 100°, vorzugsweise bei etwa Raumtemperatur (25 °C), verseift werden.

Hydrogenolytisch entfernbare Schutzgruppen (z. B. Benzyl) können z. B. durch Behandeln mit Wasserstoff oder einer Wasserstoff freisetzenden Verbindung, z. B. Ammoniumformiat, in Gegenwart eines Katalysators (z. B. eines Edelmetallkatalysators wie Palladium, zweckmäßig auf einem Träger wie Kohle) abgespalten werden. Als Lösungsmittel eignen sich dabei Lösungsmittel, wie sie üblicherweise bei Hydrierungen verwendet werden, insbesondere z. B. Alkohole wie Methanol oder Ethanol, Ether, wie Diethylether, Tetrahydrofuran (THF) und 1,4-Dioxan, oder Amide wie DMF. Die Hydrogenolyse wird in der Regel bei Temperaturen zwischen etwa 0 und 100° und Drucken zwischen etwa 1 und 200 bar, bevorzugt bei 20-30 °C und 1-10 bar durchgeführt.

Besonders bevorzugt wird als geschützte Verbindung der Formel III, d. h. als mit einer oder mehreren Schutzgruppen versehene Verbindung der Formel III, eine Verbindung der Formel IIIa oder IIIb 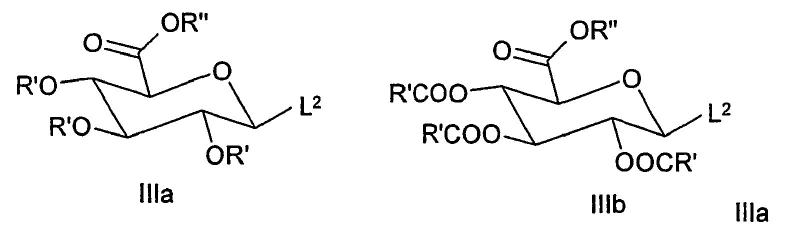
eingesetzt, worin jeder Rest R' und R" unabhängig voneinander ausgewählt ist unter H, verzweigten oder unverzweigten Alkylresten mit 1 bis 10 Kohlenstoffatomen, Aralkylresten mit 6 bis 10 Kohlenstoffatomen und Arylresten mit 6 bis 10 Kohlenstoffatomen, vorzugsweise Methyl, Ethyl, n-Proply, Isopropyl, n-Butyl, Isobutyl, tert.-Butyl, Benzyl, p-Nitrophenyl, p-Toluolyl, besonders bevorzugt Methyl, Benzyl und tert.-Butyl und insbesondere Methyl, mit der Maßgabe, dass wenigstens einer der Reste R' und R" von H verschieden ist. Vorzugsweise sind mehrere der Reste R' und R" und insbesondere alle Reste R' und R" von H verschieden. Ganz besonders bevorzugt sind Verbindungen der Formel IIIb, worin R' und R" für Methyl steht.

Besonders bevorzugt wird als geschützte Verbindung der Formel III, d. h. als mit einer oder mehreren Schutzgruppen versehene Verbindung der Formel III, daher eine Verbindung der Formel IIIc 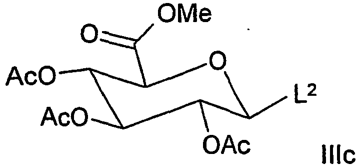 eingesetzt, worin die Abkürzung Ac für einen Acetyl-Rest steht und Me für eine Methylgruppe steht. In den Formeln IIIa, IIIb und IIIc steht L² vorzugsweise für Br.

Verbindungen der Formel III, worin L² für SR³ oder S-C(O)-R³ steht, können nach bekannten Methoden erhalten werden. Beispielsweise können sie nach der von Arie L. Gutmanet al., Synthesis 2000, 1241-1246, beschriebenen Methode oder in Analogie dazu hergestellt werden.

Ebenfalls bevorzugt wird als geschützte Verbindung der Formel III, d. h. als mit einer oder mehreren Schutzgruppen versehene Verbindung der Formel III, daher eine Verbindung der Formel IIId oder IIIe 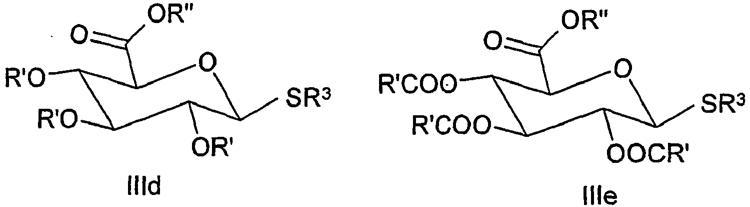 eingesetzt, worin R' und R³ wie vorstehend definiert sind und insbesondere worin in der Formel IIId R' für Benzyl und in der Formel IIIe R' für Isopropyl steht. R³ steht vorzugsweise für Ethyl oder Phenyl. Verbindungen der Formel IIId oder IIIe können beispielsweise ausgehend von Verbindungen der Formel IIIa oder IIIb und insbesondere von Verbindungen der Formel IIIc erhalten werden, insbesondere solchen, bei denen L² für Halogen steht, wobei in einem ersten Schritt ein Rest L² ungleich SR³ in einen Rest L² gleich SR³ überführt wird, z. B. durch Substitution und insbesondere durch nukleophile Substitution.

Das erfindungsgemäße Verfahren kann im Sinne einer Eintopfreaktion durchgeführt werden, d. h. auf Isolierungs- und/oder Reinigungsschritte wird so weit wie möglich verzichtet und nur das gewünschte Endprodukt, d. h. in der Regel ein erfindungsgemäßes Derivat oder ein geschütztes Derivat davon, bevorzugt ein erfindungsgemäßes Derivat und insbesondere eine Verbindung der Formel I, gereinigt und/oder isoliert. Alternativ kann nach jedem der genannten Reaktionsschritte ein Reinigungs- und/oder Isolierungsschritt durchgeführt werden. Auch gemischte Formen der vorstehend beschriebenen Verfahrensweisen sind denkbar.

Geeignete Reinigungs- und Isolierungsschritte sind dem Fachmann bekannt, z. B. aus Houben-Weyl, Methoden der organischen Chemie.

Wenn man beim wie vorstehend beschriebenen erfindungsgemäßen Verfahren gemäß Verfahrenschritt b) eine Verbindung der Formel III einsetzt, worin R1 als ganz oder teilweise mit Schutzgruppen versehenes Derivat vorliegt, jedoch von der Option unter Verfahrenschritt c) Gebrauch macht und die Schutzgruppen von R1 nicht oder nicht vollständig abspaltet, erhält man ein erfindungsgemäßes Derivat, das eine oder mehrere Schutzgruppen umfasst. Alternativ kann man ein erfindungsgemäßes Derivat, das eine oder mehrere Schutzgruppen umfasst, auch dadurch erhalten, dass man ein bei einem erfindungsgemäßen Derivat, das wenigstens eine weitere funktionelle Gruppe, vorzugsweise ausgewählt unter Hydroxy-Gruppen, zur Salzbildung befähigten Säurefunktionen und als Salz vorliegenden Säurefunktionen, aufweist, eine oder mehrere dieser funktionellen Gruppen mit einer Schutzgruppe versieht. Wie vorstehend beschriebene erfindungsgemäße Derivate, die wenigstens eine Schutzgruppe bzw. einen mit einer Schutzgruppe versehene funktionelle Gruppe aufweisen, werden im Folgenden als geschützte Derivate bezeichnet.

Unter geschützten Derivaten sind im Sinne dieser Erfindung sind daher insbesondere solche erfindungsgemäßen Derivate zu verstehen, bei denen die kovalent gebundene Säure eine oder mehrere weitere funktionelle Gruppen, insbesondere Hydroxy- und/oder Säuregruppen, aufweist, bei denen eine oder mehrere der weiteren funktionellen Gruppen wie vorstehend beschrieben geschützt, d. h. mit einer Schutzgruppe versehen ist. Weitere funktionelle Gruppen im Sinne dieser Erfindung sind die funktionellen Gruppen und insbesondere die Hydroxy- und/oder Säuregruppen der erfindungsgemäß kovalent gebundenen Säuren, welche nicht die kovalente Bindung an den N-Methyl-N-[(1S)-1-phenyt-2-((3S)-3-hydroxypyrrolidin-1-yl)-ethyl]-2,2-diphenyl-acetamid-Rest bewirken.

Beispiele für bevorzugte geschützte Derivate sind erfindungsgemäße Derivate, bei denen als weitere funktionelle Gruppen eine oder mehrere Hydroxy- und/oder eine oder mehrere Säuregruppen vorliegen, wobei die Hydroxygruppen ganz oder teilweise acetalisiert und/oder in die Säuregruppen ganz oder teilweise in Formen der Alkylester und insbesondere der Methylester vorliegen.

Bevorzugte geschützte Derivate, d. h. mit einer oder mehreren Schutzgruppen versehene Derivate, sind Verbindungen der Formel Iaa 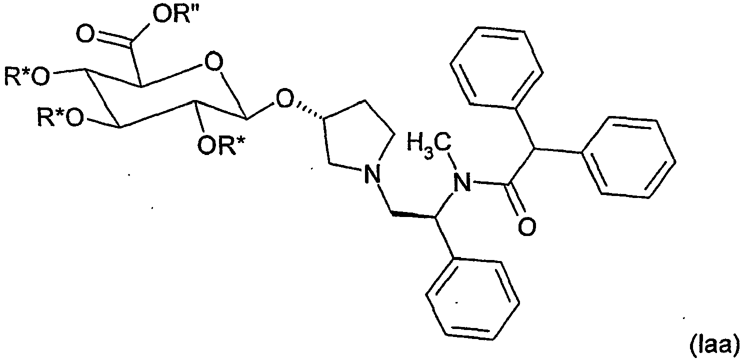 und Verbindungen der Formel Ibb, 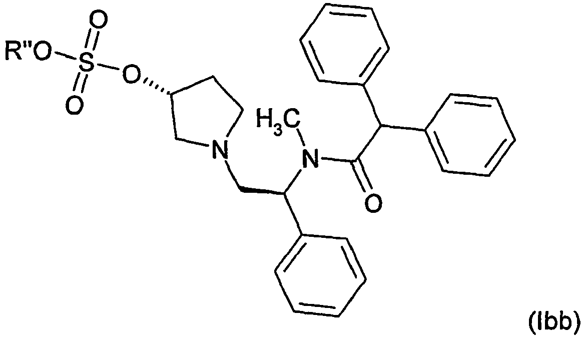 worin alle Reste R* unabhängig voneinander ausgewählt sind unter H und R'CO, und die Reste R' und R" wie vorstehend definiert sind, mit der Maßgabe, dass in der Verbindung der Formel laa und der Verbindung der Formel Ibb jeweils wenigstens einer der Reste R*, R' und R" von H verschieden ist,
sowie die Salze und Solvate davon.

Ganz besonders bevorzugte geschützte Derivate sind die Verbindungen der Formel Iaa sowie die Salze und Solvate davon.

Geschützte Derivate im Sinne dieser Erfindung sind vorzugsweise solche, bei denen alle Reste R*, R' und R" von H verschieden sind, oder, wenn zwei oder mehrere Reste, ausgewählt unter R*, R' und R", enthalten sind, solche, bei denen nur einer oder zwei der Reste R*, R' und R" gleich H sind, und bevorzugt solche, in denen nur einer der Reste R*, R' und R" gleich H ist.

Die erfindungsgemäßen Derivate wechselwirken, wie vorstehend beschrieben, intensiv mit dem Organismus, insbesondere dem enterohepatischen Kreislauf. Versuche haben gezeigt, dass auch wie vorstehend beschriebene geschützte Derivate unter physiologischen Bedingungen, z. B. durch wie vorstehend beschriebene Wechselwirkungen, ganz oder teilweise zu erfindungsgemäßen Derivaten modifiziert oder in diese umgewandelt werden können. Somit sind geschützte Derivate als gleichwirkend mit den erfindungsgemäßen Derivaten anzusehen. Somit sind auch die geschützte Derivate Gegenstand der vorliegenden Erfindung und können, wie nachfolgend beschrieben, als Wirkstoff zur Behandlung von Krankheiten und insbesondere zur Herstellung von Arzneimitteln und/oder pharmazeutischen Zubereitungen eingesetzt werden.

Bevorzugt als Prodrugs sind Derivate, worin die kovalent gebundene Säure ausgewählt ist unter zweibasigen Carbonsäuren, einbasigen Hydroxycarbonsäuren. Bevorzugte einbasige Hydroxycarbonsäuren in diesem Sinne sind Zuckersäuren.

Besonders bevorzugt als Prodrugs sind erfindungsgemäße Derivate, bei denen die kolvalent gebundene Säure unter Zuckersäuren ausgewählt ist, und bei denen eine oder mehrere der wie vorstehend beschriebenen weiteren funktionellen Gruppen eine wie vorstehend beschriebene Schutzgruppe aufweist. Besonders bevorzugt als Prodrugs sind erfindungsgemäße Derivate, bei denen die Hydroxygruppen ganz oder teilweise acetalisiert und/oder bei denen die Säuregruppen ganz oder teilweise in Formen der Alkylester und insbesondere der Methylester vorliegen.

Die Verbindungen der allegemeinen Formel I und ihre physiologisch unbedenklichen Salze können daher zur Herstellung pharmazeutischer Präparate verwendet werden, Indem man sie zusammen mit mindestens einem Träger- oder Hilfsstoff und, falls erwünscht, mit einem oder mehreren weiteren Wirkstoffen in die geeignete Dosierungsform bringt. Gegenstand der vorliegenden Erfindung ist daher ein Verfahren zur Herstellung pharmazeutischer Zusammensetzungen wie vorstehend beschrieben, und insbesondere ein Verfahren zur Herstellung pharmazeutischer Zubereitungen, wobei man wenigstens ein erfindungsgemäße Derivate und wenigstens eine weitere Verbindung, ausgewählt unter Trägern, Exzipienten, Hilfsstoffen und von erfindungsgemäßen Derivaten verschiedenen pharmazeutischen Wirkstoffen, unter Verwendung einer oder mehrerer mechanischer Verfahrensschritte in einer pharmazeutische Zusammensetzungen überführt, die als Dosierungsformen zur Verabreichung an Patienten geeignet ist. Geeignete mechanische Verfahrensschritte sind dem Fachmann bekannt und umfassen, unter anderem, Mischvorgänge, Mahlvorgänge, Lösungsvorgänge, Siebvorgänge, Homogenisieren, Trocknen, Pressen, Tablettieren, Beschichten und/oder Dragieren.

Gegenstand der vorliegenden Erfindung sind daher auch die pharmazeutischen Zusammensetzungen, die nach diesem Verfahren erhältlich sind.

Geeignete Dosierungsformen im Sinne der Erfindung sind vorzugsweise Tabletten, Dragees, Kapseln, Sirupe, Säfte, Tropfen, Suppositorien, Pflaster, Lösungen, insbesondere parenterale Lösungen, Suspensionen, Cremes, Salben, Emulsionen oder Implantate, die wenigstens ein erfindungsgemäßes Derivat und/oder ein Salz oder Solvat davon enthalten.

Gegenstand der Erfindung ist daher auch eine pharmazeutische Zubereitung, gekennzeichnet durch einen Gehalt an mindestens einem erfindungsgemäßen Derivat und/oder einem seiner Salze, insbesondere physiologisch unbedenklichen Salze, wie vorstehend beschrieben, und insbesondere wie vorstehend beschriebenen pharmazeutischen Zubereitungen zur Behandlung und/oder Prophylaxe einer oder mehrerer der vorstehend beschriebenen Erkrankungen.

Gegenstand der vorliegenden Erfindung ist auch eine pharmazeutische Zubereitung, enthaltend wenigstens ein erfindungsgemäßes Derivat und/oder ein Salz oder Solvat davon, und wenigstens einen weiteren, von den erfindungsgemäßen Derivaten und vorzugsweise auch von N-Methyl-N-[(1S)-1-phenyl-2-((3S)-3-hydroxypyrrolidin-1-yl)-ethyl]-2,2-diphenyl-acetamid verschiedenen Wirkstoff.

Gegenstand der vorliegenden Erfindung ist auch eine pharmazeutische Zubereitung, enthaltend wenigstens ein erfindungsgemäßes Derivat und/oder ein Salz oder Solvat davon, und wenigstens einen weiteren Wirkstoff, vorzugsweise einen Wirkstoff, der als Appetitzügler wirkt.

Die so erhaltenen Zubereitungen können als Arzneimittel in der Human - oder Veterinärmedizin eingesetzt werden. Als Trägersubstanzen kommen organische oder anorganische Stoffe in Frage, die sich für die enterale (z.

B. orale oder rektale) oder parenterale Applikation eignen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlenhydrate wie Lactose oder Stärke, Magnesiumstearat, Talk oder Cellulose.

Zur oralen Anwendung dienen insbesondere Tabletten, Dragees, Kapseln, Sirupe, Säfte oder Tropfen. Von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wäßsrige Lösungen, ferner Suspensionen, Emulsionen oder Implantate.

Die erfindungsgemäßs beanspruchten Wirkstoffe können auch lyophilisiert und die erhaltenen Lyophilisate z. B. zur Herstellung von Injektionspräparaten verwendet werden.

Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z. B. ein oder mehrere Vitamine, Diuretika, Antiphlogistika.

Die erfindungsgemäßsen Derivate werden in der Regel in Analogie zu anderen bekannten, für die beanspruchten Indikationen im Handel erhältlichen Präparaten verabreicht, vorzugsweise in Dosierungen zwischen etwa 1 mg und 70 mg, insbesondere zwischen 5 und 50 mg pro Dosierungseinheit. Die tägliche Dosierung liegt vorzugsweise zwischen etwa 0.02 und 30 mg/kg, insbesondere 0,2 und 0,6 mg/kg Körpergewicht.

Die spezielle Dosis für jeden einzelnen Patienten hängt jedoch von den verschiedensten Faktoren ab, beispielsweise von der Wirksamkeit der eingesetzten speziellen Verbindung, vom Alter, Körpergewicht, allgemeinem Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

### Beispiele:

### Herstellung von 6-(1-{[(2,2-Diphenyl-ethanoyl)-methyl-amino]-phenylethyl}-pyrrolidin-3-yloxy)-3,4,5-trihydroxy-tetrahydro-pyran-2-carbonsäure (Verbindung la)

a) Ein Gemisch von 2,0 g (4,434 mmol) N-Methyl-N-[(1S)-1-phenyl-2-((3S)-3-hydroxypyrrolidin-1-yl)-ethyl]-2,2-diphenyl-acetamid und 1,76 g (4,430 mmol) Glycosylhalogenid A werden in 50 ml Acetonitril vorgelegt, ein Überschuss eines Silbersalzes, z. B. Silberperchlorat oder Silbercarbonat, zugegeben und das Reaktionsgemisch bei Raumtemperatur über Nacht gerührt. Nach Abfiltrieren über Kieselgur wird das Lösungsmittel im Vakuum entfernt und der Rückstand chromatographisch gereinigt (Flash-Chromatographie an SI-60 mit Toluol/Methanol = 9:1 → 4:1 als Eluenten; R_{f} = 0.54, Toluol/MeOH 4:1) gereinigt. Man erhält 190 mg von Verbindung B.
b) 110 mg (0,151 mmol) von Verbindung B werden in 4 ml 1,4-Dioxan gelöst, mit 0,755 ml 1N Natronlauge versetzt und bei Raumtemperatur gerührt. Reaktionskontrolle mit HPLC zeigt, dass nach ca. drei Stunden Rühren die Reaktion beendet ist. Anschließend wird das Reaktionsgemisch mit 1 N Salzsäure neutralisiert und bis zur Trockene eingeengt. Der erhaltene Rückstand kann durch präparative HPLC an RP-18 gereinigt werden. Man erhält 65 mg Verbindung (Ia) (Rₜ = 31.65 min (Lichrospher 1000, RP-18, 5 µm, Gradientenelution (A:B von 99:1 bis 1:99 in 1 h; A: H₂O + 0.3 % TFA (=Trifluoressigsäure); B: CH₃CN/H₂O (80:20) + 0.3 % TFA).

Alternativ kann die Synthese als Eintopf Reaktion durchgeführt werden, bei der auf die chromatographische Aufreinigung des Reaktionsproduktes aus Schritt a) verzichtet wird. In diesem Fall wird nach beendeter Reaktion das Reaktionsgemisch filtriert und das Lösungsmittel im Vakuum entfernt.

Der erhaltene Rückstand kann anschließend, ggf. nach Aufnahme in Wasser und extrahieren mit Dichlormethan oder Methylacetat, wie in Schritt b) beschrieben, mit Natronlauge versetzt und bei Raumtemperatur gerührt werden. Die Aufarbeitung des so erhaltenen Rohprodukts an 6-(1-{[(2,2-Diphenyl-ethanoyl)-methyl-amino]-phenyl-ethyl}-pyrrolidin-3-yloxy)-3,4,5-trihydroxy-tetrahydro-pyran-2-carbonsäure kann die unter Schritt b) beschrieben erfolgen.

### Herstellung von Schwefelsäuremono-{1-[2-(diphenylacetyl-methyl-amino)-2-phenyl-ethyl]-pyrrolidin-3-yl}ester (Ib)

0,9 Gramm (2,0 mmol) N-Methyl-N-[(1S)-1-phenyl-2-((3S)-3-hydroxypyrrolidin-1-yl)-ethyl]-2,2-diphenyl-acetamid werden in 10 ml Dichlormethan vorgelegt und mit 150 µl (2,0 mmol) Chlorsulfonsäure versetzt. Nach einer Reaktionszeit von zwei Tagen wird das Lösungsmittel abdestilliert, der erhaltene Rückstand mehrfach mit Aceton vertrieben und das überstehende Aceton abdekantiert, die so erhaltenen Kristalle im Vakuum abfiltriert und an Luft getrocknet. Nach Trocknen an Luft erhält man 740 mg (74,3 % der Theorie) kristallinen Feststoff von Verbindung (Ib) mit einem Schmelzpunkt von 268 °C.

### Herstellung von N-{2-[(3S)-3-acetoxy-1-pyrrolidinyl]-(1S)-1-phenylethyl}-2,2-diphenyl-N-methylacetamid (Verbindung Ic)

5,0 Gramm (11,0 mmol) N-Methyl-N-[(1S)-1-phenyl-2-((3S)-3-hydroxypyrrolidin-1-yl)-ethyl]-2,2-diphenyl-acetamid, Hydrochlorid, werden mit 30 ml Essigsäureanhydrid und 15 ml Triethylamin versetzt und zwei Stunden auf dem Dampfbad erwärmt. Anschließend wird das Reaktionsgemisch bis zum Rückstand eingedampft, der Rückstand in Ether aufgenommen und mit Bicarbonat-Lösung gewaschen. Die organische Phase wird anschließend getrocknet und das Lösungsmittel abdestilliert. Der erhaltene Rückstand wird durch Säulenchromatographie an Kieselgel mit Diethylether/Methanol (99:1) als Eluenten gereinigt. Das so erhaltene Rohprodukt von N-{2-[(3S)-3-acetoxy-1-pyrrolidinyl]-(1S)-1-pheny)ethy)}-2,2-diphenyl-N-methylacetamid kann durch Aufnehmen in Diethylether, Ausfällen durch Versetzen mit etherischer HCl, Abfiltrieren der erhaltenen Kristalle im Vakuum, Waschen der Kristalle mit Ether und Trocknen an Luft weiter gereinigt werden (R_{f} = 0.6 (DC auf Kieselgel 6 F₂₅₄ mit Dichlormethan/Methanol (8:2) als Eluenten)).

## Patentansprüche

1. Derivate von N-Methyl-N-[(1S)-1-phenyl-2-((3S)-3-hydroxypyrrolidin-1-yl)-ethyl]-2,2-diphenyl-acetamid mit wenigstens einer gebundenen Säure, und die Salze, Solvate und Prodrugs davon, **dadurch gekennzeichnet, dass** die Säure ausgewählt ist unter zweibasigen Carbonsäuren, einbasigen Hydroxycarbonsäuren und wenigstens zweibasigen anorganischen Sauerstoffsäuren und über die 3-Hydroxypyrrolidin-Gruppe des N-Methyl-N-[(1S)-1-phenyl-2-((3S)-3-hydroxypyrrolidin-1-yl)-ethyl]-2,2-diphenyl-acetamids kovalent gebunden ist, wobei es sich bei den Prodrugs um Alkylester von Derivaten, die wenigstens eine freie Säuregruppe aufweisen, oder Ester von Derivaten, die wenigstens eine freie Hydroxy-Gruppe aufweisen, handelt.

2. Derivat nach Anspruch 1, **dadurch gekennzeichnet, dass** die Säure ausgewählt ist unter physiologisch verträglichen Säuren.

3. Derivat nach Anspruch 1 oder 2, **dadurch gekennzeichnet, dass** das Derivat wenigstens eine zur Salzbildung befähigte Säurefunktion oder eine als Salz vorliegende Säurefunktion aufweist.

4. Derivat nach einem der Ansprüche 1 bis 3, **dadurch gekennzeichnet dass** die einbasige Hydroxycarbonsäure ausgewählt ist unter Zuckersäuren.

5. Derivat nach Anspruch 4, **dadurch gekennzeichnet, dass** die Zuckersäure Glucuronsäure ist.

6. Derivat nach einem der Ansprüche 1 bis 3, **dadurch gekennzeichnet, dass** die zweibasige anorganische Sauerstoffsäure Schwefelsäure ist.

7. Derivat nach einem der Ansprüche 1 bis 6, ausgewählt unter 6-(1-{[(2,2-Diphenyl-ethanoyl)-methyl-amino]-phenyl-ethyl}-pyrrolidin-3-yloxy)-3,4,5-trihydroxy-tetrahydro-pyran-2-carbonsäure und Schwefelsäuremono-{1-[2-(diphenylacetyl-methyl-amino)-2-phenyl-ethyl]-pyrrolidin-3-yl}ester.

8. Derivat nach einem der Ansprüche 1 bis 7 und/oder ein Salz, ein Solvat oder Prodrug davon zur Anwendung als Arzneimittel, wobei es sich bei dem Prodrug um einen Alkylester eines Derivats, das wenigstens eine freie Säuregruppe aufweist, oder einen Ester eines Derivats, das wenigstens eine freie Hydroxy-Gruppe aufweist, handelt.

9. Derivat nach einem der Ansprüche 1 bis 7 und/oder ein Salz oder Solvat davon zur Anwendung als Opiat-Rezeptor-Agonist.

10. Derivat nach einem der Ansprüche 1 bis 7 und/oder ein Salz oder Solvat davon zur Anwendung als Opiat-Rezeptor-Agonist zur Prävention und/oder Behandlung von Krankheiten.

11. Derivat nach Anspruch 10, **dadurch gekennzeichnet, dass** die Krankheiten ausgewählt sind unter funktionellen gastrointestinalen Erkrankungen, entzündlichen und nicht entzündlichen Erkrankungen des Gastrointestinaltraktes, entzündlichen und nicht entzündlichen Erkrankungen des Harnwegs, Ess- und Verdauungsstörungen und Krankheiten, die mit starken Schmerzen oder Schmerzzuständen verbunden sind.

12. Derivat gemäß einem der Ansprüche 1 bis 7 und/oder ein Salz oder Solvat davon zur Anwendung als Arzneimittel zur Prophylaxe und/oder Bekämpfung von Krankheiten.

13. Derivat nach Anspruch 12, **dadurch gekennzeichnet, dass** die Krankheiten ausgewählt sind unter den in Anspruch 11 genannten Krankheiten.

14. Derivat gemäß einem der Ansprüche 1 bis 7 und/oder ein Salz oder Solvat davon zur Anwendung als Arzneimittel zur Prophylaxe und/oder Behandlung von Schmerzen, Schmerzzuständen, Ohrenschmerzen, Augenschmerzen, Entzündungen, Ileus, funktionellen gastrointestinalen Erkrankungen, funktionellen Darmerkrankungen, entzündlichen Darmerkrankungen, Reizdarm, Reizblase, chronischen Motilitätsstörungen, Dyspepsie, Neuropathie, Adipositas, Bulimie, Fettsucht, Kachexie, Anorexie, Dysorexie, Dysponderosis, Gastroparese und Stenosen des Gastrointestinaltraktes.

15. Derivat gemäß einem der Ansprüche 1 bis 7 und/oder ein Salz oder Solvat davon zur Anwendung als Arzneimittel in Kombination mit einem oder mehreren Pharmazeutika, die als Appetitzügler wirken.

16. Verfahren zu Herstellung einer pharmazeutischen Zusammensetzung, **dadurch gekennzeichnet dass** man wenigstens ein Derivat gemäß einem der Ansprüche 1 bis 7 und wenigstens einer weitere Verbindung, ausgewählt unter Trägem, Exzipienten, Hilfsstoffen und von Derivaten gemäß einem der Ansprüche 1 bis 7 verschiedenen pharmazeutischen Wirkstoffen, unter Verwendung einer oder mehrerer mechanischer Verfahrensschritte in eine pharmazeutische Zusammensetzung überführt, die als Dosierungsform zur Verabreichung an Patienten geeignet ist.

17. Pharmazeutische Zusammensetzung, **dadurch gekennzeichnet, dass** sie wenigstens ein Derivat gemäß einem der Ansprüche 1 bis 7 enthält.

18. Pharmazeutische Zusammensetzung nach Anspruch 17, **dadurch gekennzeichnet, dass** sie wenigstens einen weiteren pharmazeutischen Wirkstoff enthält.

19. Pharmazeutische Zusammensetzung nach Anspruch 18, **dadurch gekennzeichnet, dass** der weitere Wirkstoff ausgewählt ist unter Phenylpropanolamin, Cathin, Sibutramin, Amfepramon, Ephedrin und Norpseudoephedrin.

20. Verfahren zur Herstellung eines Derivats gemäß einem der Ansprüche 1 bis 7, wobei man
a) eine Verbindung der Formel II 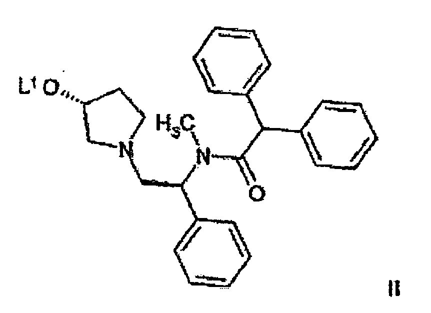 worin
L¹ H oder ein Metallion bedeutet;
b) mit einer Verbindung der Formel III,
R¹-L² III
worin
L² für eine Abgangsgruppe steht, und
R¹ ausgewählt ist unter substituierten oder unsubstituierten Acylresten mit 1 bis 12 Kohlenstoffatomen, Alkylresten, die durch Entfernung einer Hydroxygruppe von Polyhydroxymonocarbonsäuren abgeleitet sind, Sulfonsäuregruppen, Phosphonsäuregruppen und Nitrogruppen, oder, wenn R¹ zusätzlich zu der Gruppe L² eine oder mehrere funktionelle Gruppen aufweist, ein ganz oder teilweise mit Schutzgruppen versehenes Derivat von R¹ umsetzt,
c) gegebenenfalls enthaltene Schutzgruppen abspaltet, gegebenenfalls die Verbindung der Formel I isoliert,
und gegebenenfalls
d) die erhaltene Verbindung der Formel I durch Behandeln mit einer Säure oder einer Base in eines ihrer Salze überführt und gegebenenfalls das Salz isoliert.

## Claims

1. Derivatives of N-methyl-N-[(1S)-1-phenyl-2-((3S)-3-hydroxypyrrolidine-1-yl)-ethyl]-2,2 diphenyl-acetamide with at least one bonded acid and the salts, solvates and prodrugs thereof, **characterized in that** said acid is selected from dibasic carboxylic acids, hydroxymonobasic carboxylic acids and at least dibasic inorganic oxygen acids and being covalently bonded via the 3-hydroxypyrrolidine group of the N-methyl-N-[(1S)-1-phenyl-2-((3S)-3-hydroxypyrrolidine-1-yl)-ethyl]-2,2-diphenyl-acetamide, wherein said prodrugs are alkyl esters of derivatives having at least one free acid group or esters of derivatives having at least one free hydroxy group.

2. Derivative according to claim 1, **characterized in that** the acid is selected from physiologically acceptable acids.

3. Derivative according to claim 1 or 2, **characterized in that** the derivative has at least one acid function that is capable of salt formation or is a salt.

4. Derivative according to any one of claims 1 to 3, **characterized in that** the monobasic hydroxy carboxylic acid is selected from sugar acids.

5. Derivative according to claim 4, **characterized in that** the sugar acid is glucuronic acid.

6. Derivative according to claim 1 to 3, **characterized in that** the at least dibasic inorganic oxygen acid is sulfuric acid.

7. Derivative according to any one of claims 1 to 6, selected from 6-(1-{[(2,2-diphenyl-ethanoyl)-methyl-amino]-phenyl-ethyl}-pyrrolidine-3-yloxy)-3,4,5,-trihydroxy-tetrahydro-pyran-2-carboxylic acid and sulfuric acid-{1-[2-(diphenylacetyl-methyl-amino)-2-phenyl-ethyl]-pyrrolidin-3-yl} ester.

8. Derivative according to any one of claims 1 to 7 and/or a salt, solvate or prodrug thereof for use as a drug, wherein the prodrug is an alkyl ester of a derivative having at least one free acid group or an ester of a derivative having at least one free hydroxy group.

9. Derivative according to any one of claims 1 to 7 and/or a salt or solvate thereof for use as an opiate receptor agonist.

10. Derivative according to any one of claims 1 to 7 and/or a salt or solvate thereof for use as an opiate receptor agonist to prevent and/or treat diseases.

11. Derivative according to claim 10, **characterized in that** the disease is selected from functional gastrointestinal diseases, inflammatory and non-inflammatory diseases of the gastrointestinal tract, inflammatory and non-inflammatory diseases of the urinary system, eating disorders dyspepsia and diseases being associated with pain or pain conditions.

12. Derivative according to any one of claims 1 to 7 and/or a salt or solvate thereof for use as a drug for the prophylaxis and/or treatment of diseases.

13. Derivative according to claim 12, **characterized in that** the disease is selected from the diseases listed in claim 11.

14. Derivative according to any one of claims 1 to 7 and/or a salt or solvate thereof for use as a drug for the prophylaxis and/or treatment of pain, pain conditions, ear ache, eye ache, inflammation, ileus, functional gastrointestinal diseases, functional bowel diseases, inflammatory bowel diseases, irritable bowel syndrome, irritable bladder syndrome, chronic motility disorder, dyspepsia, peripheral neuropathy, adiposity, bulimia nervosa, obesity, cachexia, anorexia, dysorexia, dysponderosis, gastroparesis and stenosis of the gastrointestinal tract.

15. Derivative according to any one of claims 1 to 7 and/or a salt or solvate thereof for use as a drug in combination with one or more pharmaceuticals that are anorectics.

16. Method for the manufacture of a pharmaceutical composition **characterized in that** at least one derivative according to any one of claims 1 to 7 and at least one other compound selected from carriers, excipients, auxiliaries and pharmaceutical agents different from the derivatives according to any one of claims 1 to 7 are converted into a pharmaceutical composition that is a dosage type suitable for administration to patients by using one or more mechanical method steps.

17. Pharmaceutical composition **characterized in that** it comprises at least one derivative according to any one of claims 1 to 7.

18. Pharmaceutical composition according to claim 17 **characterized in that** it contains at least one additional active pharmaceutical agent.

19. Pharmaceutical composition according to claim 18 **characterized in that** the active pharmaceutical compound is selected from phenylpropanolamine, cathine, sibutramine, amfepramone, ephedrine and norpseudoephedrine.

20. Method for the manufacture of a derivative according to any one of claims 1 to 7, wherein
a) a compound of formula II 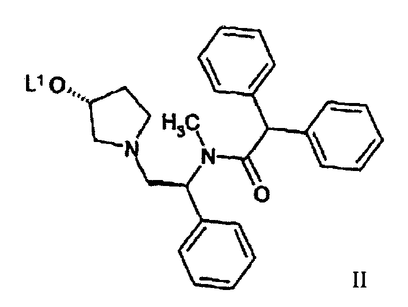 wherein L¹ is H or a metal ion; is reacted with
b) a compound of formula III,
R¹-L² III
wherein
L² represents a leaving group, and
R' is selected from substituted or unsubstituted acyl moieties with 1 to 12 carbon atoms, alkyl moieties derived from polyhydroxy monocarboxylic acids by removal of a hydroxyl group, sulfonic acid groups, phosphoric acid groups and nitro groups, or, if R¹ includes in addition to the L² group one or more functional groups, a derivative of R¹ completely or partially provided with protective groups,
c) contained protective groups are optionally cleared off, the compound of formula I is optionally isolated,
and optionally
d) the obtained compound of formula I is converted into one of its salts by treatment with an acid or base and the salt is optionally isolated.

## Revendications

1. Dérivés de N-méthyl-N-[(1S)-1-phényl-2-((3S)-3-hydroxypyrrolidine-1-yl)-éthyl]-2,2-diphényl-acétamide avec au moins un acide lié, et les sels, produits de solvatation et prodrogues de ces derniers, **caractérisés en ce que** l'acide est choisi parmi les acides carboxyliques dibasiques, les acides hydroxycarboxyliques monobasiques et les oxyacides inorganiques au moins dibasiques, et est lié d'une manière covalente par l'intermédiaire du groupe 3-hydroxypyrrolidine du N-méthyl-N-[(1S)-1-phényl-2-((3S)-3-hydroxypyrrolidine-1-yl)-éthyl]-2,2-diphényl-acétamide, où, pour ce qui concerne les prodrogues, il s'agit d'esters alkyliques de dérivés qui comprennent au moins un groupe acide libre, ou d'esters de dérivés qui comprennent au moins un groupe hydroxy libre.

2. Dérivé selon la revendication 1, **caractérisé en ce que** l'acide est choisi parmi les acides physiologiquement tolérés.

3. Dérivé selon la revendication 1 ou 2, **caractérisé en ce que** le dérivé comprend au moins une fonction acide salifiable, ou une fonction acide présente sous la forme d'un sel.

4. Dérivé selon l'une des revendications 1 à 3, **caractérisé en ce que** l'acide hydroxycarboxylique monobasique est choisi parmi les alcools de sucres.

5. Dérivé selon la revendication 4, **caractérisé en ce que** l'alcool de sucre est l'acide glucuronique.

6. Dérivé selon l'une des revendications 1 à 3, **caractérisé en ce que** l'oxyacide inorganique dibasique est l'acide sulfurique.

7. Dérivé selon l'une des revendications 1 à 6, choisi parmi l'acide 6-(1-{[(2,2-diphényl-éthanoyl)-méthyl-amino]-phényl-éthyl}-pyrrolidine-3-yloxy)-3,4,5-trihydroxy-tétrahydro-pyranne-2-carboxylique et l'ester mono-{1-[2-(diphénylacétyl-méthyl-amino)-2-phényl-éthyl]-pyrrolidine-3-ylique} de l'acide sulfurique.

8. Dérivé selon l'une des revendications 1 à 7, et/ou sel, produit de solvatation ou prodrogue de ce dérivé, pour utilisation en tant que médicament, dans lequel, pour ce qui concerne la prodrogue, il s'agit d'un ester alkylique d'un dérivé comprenant au moins un groupe acide libre ou d'un ester d'un dérivé comprenant au moins un groupe hydroxy libre.

9. Dérivé selon l'une des revendications 1 à 7, et/ou sel ou produit de solvatation de ce dérivé, pour utilisation en tant qu'agoniste des récepteurs opiacés.

10. Dérivé selon l'une des revendications 1 à 7 et/ou sel ou produit de solvatation de ce dérivé, pour utilisation en tant qu'agoniste des récepteurs opiacés pour la prévention et/ou le traitement de maladies.

11. Dérivé selon la revendication 10, **caractérisé en ce que** les maladies sont choisies parmi les affections gastro-intestinales fonctionnelles, les affections inflammatoires et non inflammatoires du tractus gastro-intestinal, les affections inflammatoires et non inflammatoires des voies urinaires, les troubles et maladies de l'alimentation et de la digestion, qui sont associés à de fortes douleurs ou à des états douloureux intenses.

12. Dérivé selon l'une des revendications 1 à 7 et/ou sel ou produit de solvatation de ce dérivé, pour utilisation en tant que médicament pour la prophylaxie de maladies et/ou la lutte contre ces dernières.

13. Dérivé selon la revendication 12, **caractérisé en ce que** les maladies sont choisies parmi les maladies mentionnées dans la revendication 11.

14. Dérivé selon l'une des revendications 1 à 7 et/ou sel ou produit de solvatation de ce dérivé, pour utilisation en tant que médicament pour la prophylaxie et/ou le traitement des douleurs, des états douloureux, des otalgies, des ophtalmalgies, des inflammations, de l'iléus, des affections gastro-intestinales fonctionnelles, des affections intestinales fonctionnelles, des affections intestinales inflammatoires, du côlon irritable, de la vessie irritable, des troubles chroniques de la motilité, de la dyspepsie, de la neuropathie, de l'adiposité, de la boulimie, de l'obésité, de la cachexie, de l'anorexie, de la dysorexie, de la dyspondérose, de la gastroparésie et des sténoses du tractus gastro-intestinal.

15. Dérivé selon l'une des revendications 1 à 7 et/ou sel ou produit de solvatation de ce dérivé, pour utilisation en tant que médicament en combinaison avec un ou plusieurs produits pharmaceutiques agissant comme des anorexiants.

16. Procédé de fabrication d'une composition pharmaceutique, **caractérisé en ce qu'**on convertit au moins un dérivé selon l'une des revendications 1 à 7 et au moins un composé supplémentaire choisi parmi les supports, les excipients, les adjuvants et les principes actifs pharmaceutiques différents des dérivés selon l'une des revendications 1 à 7, par utilisation d'une ou plusieurs étapes de procédés mécaniques, en une composition pharmaceutique qui convient en tant que forme posologique pour administration à des patients.

17. Composition pharmaceutique, **caractérisée en ce qu'**elle contient au moins un dérivé selon l'une des revendications 1 à 7.

18. Composition pharmaceutique selon la revendication 17, **caractérisée en ce qu'**elle contient au moins un principe actif pharmaceutique supplémentaire.

19. Composition pharmaceutique selon la revendication 18, **caractérisée en ce que** le principe actif supplémentaire est choisi parmi la phénylpropanolamine, la cathine, la sibutramine, l'amfépramon, l'éphédrine et la norpseudoéphédrine.

20. Procédé de préparation d'un dérivé selon l'une des revendications 1 à 7, dans lequel on fait réagir
a) un composé de formule II 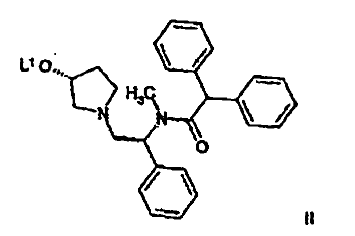 dans laquelle L¹ est H ou un ion métallique ;
b) avec un composé de formule III
R¹-L² III
dans laquelle
L² est un groupe partant, et
R¹ est choisi parmi les radicaux acyle substitués ou non substitués ayant 1 à 12 atomes de carbone, les radicaux alkyle qui dérivent d'acides polyhydroxymonocarboxyliques par élimination d'un groupe hydroxy, les groupes acide sulfonique, les groupes acide phosphonique et les groupes nitro, ou, quand R¹ comprend, en plus du groupe L², un ou plusieurs groupes fonctionnels, un dérivé de R¹ pourvu en totalité ou en partie de groupes protecteurs,
c) on dissocie les groupes protecteurs éventuellement présents, éventuellement on isole le composé de formule I,
et éventuellement
d) on convertit en l'un de ses sels le composé obtenu de formule I, par traitement avec un acide ou une base, et éventuellement on isole le sel.
